# EUROPEAN PATENT APPLICATION

(11) **EP 1 471 145 A2**
(43) Date of publication of application: **27.10.2004**
(21) Application number: 04015832.1
(22) Date of filing: 23.03.1994
(51) Int. Cl.: C12N 15/31, C12N 15/32, C12N 15/82, A01H 5/00, A01N 63/00, C12P 1/04, C12N 1/21, C12Q 1/68, C12P 21/08

(54) **Pesticipal proteins and strains**

(30) Priority: 25.03.1993 US 37057
(62) Divisional of application: 94911683.4
(71) Applicant: Syngenta Participations AG, 4058 Basel (CH)
(72) Inventor: Koziel, Michael Gene, Raleigh, NC 27613 (US); Mullins, Martha Alice, Apex, NC 27502 (US); Nye, Gordon James, Morrisville, NC 27560 (US); Warren, Gregory Wayne, Research Triangle Park, NC 27709 (US)
(74) Representative: Bastian, Werner Maria

(57) **Abstract**

The present invention is drawn to pesticidal strains and proteins. *Bacillus* strains which are capable of producing pesticidal proteins and auxiliary proteins during vegetative growth are provided. Also provided are the purified proteins, nucleotide sequences encoding the proteins and methods for using the strains, proteins and genes for controlling pests.

SEQUENCE LISTING

(1) GENERAL INFORMATION:

(i) APPLICANT:
(A) NAME: CIBA-GEIGY AG
(B) STREET: Klybeckstrasse 141
(C) CITY: Basle
(E) COUNTRY: Switzerland
(F) POSTAL CODE (ZIP): CH-4002
(G) TELEPHONE: (061) 696 11 11
(H) TELEFAX: (061) 696 79 76

(A) NAME: Gregory W. Warren
(B) STREET: 324 Bond Lake Drive
(C) CITY: Cary
(D) STATE: NC
(E) COUNTRY: USA
(F) POSTAL CODE (ZIP): 27513

(A) NAME: Michael G. Koziel
(B) STREET: 509 Carolyn Court
(C) CITY: Cary
(D) STATE: NC
(E) COUNTRY: USA
(F) POSTAL CODE (ZIP): 27511

(A) NAME: Martha A. Mullins
(B) STREET: 104 Countrybrook Lane
(C) CITY: Youngsville
(D) STATE: NC
(E) COUNTRY: USA
(F) POSTAL CODE (ZIP): 27596

(A) NAME: Gordon J. Nye
(B) STREET: 1001 Bray Court
(C) CITY: Apex
(D) STATE: NC
(E) COUNTRY: USA
(F) POSTAL CODE (ZIP): 27502

(A) NAME: Brian Carr
(B) STREET: 110 D Lady's Slipper Ct.
(C) CITY: Raleigh
(D) STATE: N.C.
(E) COUNTRY: U.S.A.
(F) POSTAL CODE (ZIP): 27606

(A) NAME: Nalini Manaj Desai
(B) STREET: 107 Silverwood Lane
(C) CITY: Cary
(D) STATE: N.C.
(E) COUNTRY: U.S.A.
(F) POSTAL CODE (ZIP): 27511

(A) NAME: N. Kristy Kostichka
(B) STREET: 5017 Wineberry Dr.
(C) CITY: Durham
(D) STATE: NC

(E) COUNTRY: USA
(F) POSTAL CODE (ZIP): 27713

(ii) TITLE OF INVENTION: Novel Pesticidal Proteins and Strains

(iii) NUMBER OF SEQUENCES: 18

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: US 08/037,057
(B) FILING DATE: 25-MAR-1993

(2) INFORMATION FOR SEQ ID NO: 1:

(i) SEQUENCE CHARACTERISTICS:
(A) LENGTH: 6106 base pairs
(B) TYPE: nucleic acid
(C) STRANDEDNESS: single
(D) TOPOLOGY: linear

(ii) MOLECULE TYPE: DNA (genomic)

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: CDS
(B) LOCATION: 1082..1810
(D) OTHER INFORMATION: /product= "VIP-2"
/label= ORF-1

(ix) FEATURE:
(A) NAME/KEY: CDS
(B) LOCATION: 1925..2470
(D) OTHER INFORMATION: /product= "VIP-2"
/label= ORF-2

(xi) SEQUENCE DESCRIPTION: SEQ ID NO: 1:

ATCGATACAA TGTTGTTTTA CTTAGACCGG TAGTCTCTGT AATTTGTTTA ATGCTATATT 60

CTTTACTTTG ATACATTTTA ATAGCCATTT CAACCTTATC AGTATGTTTT TGTGGTCTTC 120

CTCCTTTTTT TCCACGAGCT CTAGCTGCGT TTAATCCTGT TTTGGTACGT TCGCTAATAA 180

TATCTCTTTC TAATTCTGCA ATACTTGCCA TCATTCGAAA GAAGAATTTC CCCATAGCAT 240

TAGAGGTATC AATGTTGTCA TGAATAGAAA TAAAATCTAC ACCTAGCTCT TTGAATTTTT 300

CACTTAACTC AATTAGGTGT TTTGTAGAGC GAGAAATTCG ATCAAGTTTG TAAACAACTA 360

TCTTATCGCC TTTACGTAAT ACTTTTAGCA ACTCTTCGAG TTGAGGGCGC TCTTTTTTTA 420

TTCCTGTTAT TTTCTCCTGA TATAGCCTTT CTACACCATA TTGTTGCAAA GCATCTATTT 480

GCATATCGAG ATTTTGTTCT TCTGTGCTGA CACGAGCATA ACCAAAAATC AAATTGGTTT 540

CACTTCCTAT CTAAATATAT CTATTAAAAT AGCACCAAAA ACCTTATTAA ATTAAAATAA 600

GGAACTTTGT TTTTGGATAT GGATTTTGGT ACTCAATATG GATGAGTTTT TAACGCTTTT 660

GTTAAAAAAC AAACAAGTGC CATAAACGGT CGTTTTTGGG ATGACATAAT AAATAATCTG 720

TTTGATTAAC CTAACCTTGT ATCCTTACAG CCCAGTTTTA TTTGTACTTC AACTGACTGA 780

ATATGAAAAC AACATGAAGG TTTCATAAAA TTTATATATT TTCCATAACG GATGCTCTAT 840

CTTTAGGTTA TAGTTAAATT ATAAGAAAAA AACAAACGGA GGGAGTGAAA AAAAGCATCT 900

TCTCTATAAT TTTACAGGCT CTTTAATAAG AAGGGGGGAG ATTAGATAAT AAATATGAAT 960

ATCTATCTAT AATTGTTTGC TTCTACAATA ACTTATCTAA CTTTCATATA CAACAACAAA 1020

ACAGACTAAA TCCAGATTGT ATATTCATTT TCAGTTGTTC CTTTATAAAA TAATTTCATA 1080

A ATG AAA AGA ATG GAG GGA AAG TTG TTT ATG GTG TCA AAA AAA TTA 1126
Met Lys Arg Met Glu Gly Lys Leu Phe Met Val Ser Lys Lys Leu
1               5                   10                  15

CAA GTA GTT ACT AAA ACT GTA TTG CTT AGT ACA GTT TTC TCT ATA TCT 1174
Gln Val Val Thr Lys Thr Val Leu Leu Ser Thr Val Phe Ser Ile Ser
                20                  25                  30

TTA TTA AAT AAT GAA GTG ATA AAA GCT GAA CAA TTA AAT ATA AAT TCT 1222
Leu Leu Asn Asn Glu Val Ile Lys Ala Glu Gln Leu Asn Ile Asn Ser
            35                  40                  45

CAA AGT AAA TAT ACT AAC TTG CAA AAT CTA AAA ATC ACT GAC AAG GTA 1270
Gln Ser Lys Tyr Thr Asn Leu Gln Asn Leu Lys Ile Thr Asp Lys Val
        50                  55                  60

GAG GAT TTT AAA GAA GAT AAG GAA AAA GCG AAA GAA TGG GGG AAA GAA 1318
Glu Asp Phe Lys Glu Asp Lys Glu Lys Ala Lys Glu Trp Gly Lys Glu
    65                  70                  75

AAA GAA AAA GAG TGG AAA CTA ACT GCT ACT GAA AAA GGA AAA ATG AAT 1366
Lys Glu Lys Glu Trp Lys Leu Thr Ala Thr Glu Lys Gly Lys Met Asn
80                  85                  90                  95

AAT TTT TTA GAT AAT AAA AAT GAT ATA NAG ACA AAT TAT AAA GAA ATT 1414
Asn Phe Leu Asp Asn Lys Asn Asp Ile Xaa Thr Asn Tyr Lys Glu Ile
                100                 105                 110

ACT TTT TCT ATG GCA GGC TCA TTT GAA GAT GAA ATA AAA GAT TTA AAA 1462
Thr Phe Ser Met Ala Gly Ser Phe Glu Asp Glu Ile Lys Asp Leu Lys
            115                 120                 125

GAA ATT GAT AAG ATG TTT GAT AAA ACC AAT CTA TCA AAT TCT ATT ATC 1510
Glu Ile Asp Lys Met Phe Asp Lys Thr Asn Leu Ser Asn Ser Ile Ile
        130                 135                 140

ACC TAT AAA AAT GTG GAA CCG ACA ACA ATT GGA TTT AAT AAA TCT TTA 1558
Thr Tyr Lys Asn Val Glu Pro Thr Thr Ile Gly Phe Asn Lys Ser Leu
    145                 150                 155

ACA GAA GGT AAT ACG ATT AAT TCT GAT GCA ATG GCA CAG TTT AAA GAA 1606
Thr Glu Gly Asn Thr Ile Asn Ser Asp Ala Met Ala Gln Phe Lys Glu
160                 165                 170                 175

CAA TTT TTA GAT AGG GAT ATT AAG TTT GAT AGT TAT CTA GAT ACG CAT 1654
Gln Phe Leu Asp Arg Asp Ile Lys Phe Asp Ser Tyr Leu Asp Thr His
                180                 185                 190

TTA ACT GCT CAA CAA GTT TCC AGT AAA GAA AGA GTT ATT TTG AAG GTT 1702
Leu Thr Ala Gln Gln Val Ser Ser Lys Glu Arg Val Ile Leu Lys Val
            195                 200                 205

ACG GTT CCG AGT GGG AAA GGT TCT ACT ACT CCA ACA AAA GCA GGT GTC 1750
Thr Val Pro Ser Gly Lys Gly Ser Thr Thr Pro Thr Lys Ala Gly Val
        210                 215                 220

ATT TTA AAT AAT AGT GAA TAC AAA ATG CTC ATT GAT AAT GGG TAT ATG 1798
Ile Leu Asn Asn Ser Glu Tyr Lys Met Leu Ile Asp Asn Gly Tyr Met
    225                 230                 235

GTC CAT GTA GAT TAAGGTATCA AAAGTGGTGA AAAAAGGGGG TGGAGTGCCT 1850
Val His Val Asp
240

TACAAATTGA AGGGACTTTA AAAAAGAGTC TTGACTTTAA AAATGATATA AATGCTGAAG 1910

CGCATAGCTG GGGT ATG AAG AAT TAT GAA GAG TGG GCT AAA GAT TTA ACC 1960
Met Lys Asn Tyr Glu Glu Trp Ala Lys Asp Leu Thr
1 5 10

GAT TCG CAA AGG GAA GCT TTA GAT GGG TAT GCT AGG CAA GAT TAT AAA 2008
Asp Ser Gln Arg Glu Ala Leu Asp Gly Tyr Ala Arg Gln Asp Tyr Lys
15 20 25

GAA ATC AAT AAT TAT TTA AGA AAT CAA GGC GGA AGT GGA AAT GAA AAA 2056
Glu Ile Asn Asn Tyr Leu Arg Asn Gln Gly Gly Ser Gly Asn Glu Lys
30 35 40

CTA GAT GCT CAA ATA AAA AAT ATT TCT GAT GCT TTA GGG AAG AAA CCA 2104
Leu Asp Ala Gln Ile Lys Asn Ile Ser Asp Ala Leu Gly Lys Lys Pro
45 50 55 60

ATA CCG GAA AAT ATT ACT GTG TAT AGA TGG TGT GGC ATG CCG GAA TTT 2152
Ile Pro Glu Asn Ile Thr Val Tyr Arg Trp Cys Gly Met Pro Glu Phe
65 70 75

GGT TAT CAA ATT AGT GAT CCG TTA CCT TCT TTA AAA GAT TTT GAA GAA 2200
Gly Tyr Gln Ile Ser Asp Pro Leu Pro Ser Leu Lys Asp Phe Glu Glu
80 85 90

CAA TTT TTA AAT ACA ATC AAA GAA GAC AAA GGA TAT ATG AGT ACA AGC 2248
Gln Phe Leu Asn Thr Ile Lys Glu Asp Lys Gly Tyr Met Ser Thr Ser
95 100 105

TTA TCG AGT GAA CGT CTT GCA GCT TTT GGA TCT AGA AAA ATT ATA TTA 2296

Leu Ser Ser Glu Arg Leu Ala Ala Phe Gly Ser Arg Lys Ile Ile Leu
    110                 115                 120

CGA TTA CAA GTT CCG AAA GGA AGT ACG GGT GCG TAT TTA AGT GCC ATT     2344
Arg Leu Gln Val Pro Lys Gly Ser Thr Gly Ala Tyr Leu Ser Ala Ile
125                 130                 135                 140

GGT GGA TTT GCA AGT GAA AAA GAG ATC CTA CTT GAT AAA GAT AGT AAA     2392
Gly Gly Phe Ala Ser Glu Lys Glu Ile Leu Leu Asp Lys Asp Ser Lys
                145                 150                 155

TAT CAT ATT GAT AAA GTA ACA GAG GTA ATT ATT AAA GGT GTT AAG CGA     2440
Tyr His Ile Asp Lys Val Thr Glu Val Ile Ile Lys Gly Val Lys Arg
            160                 165                 170

TAT GTA GTG GAT GCA ACA TTA TTA ACA AAT TAAGGAGATG AAAAATATGA     2490
Tyr Val Val Asp Ala Thr Leu Leu Thr Asn
        175                 180

AGAAAAAGTT AGCAAGTGTT GTAACGTGTA CGTTATTAGC TCCTATGTTT TTGAATGGAA     2550

ATGTGAATGC TGTTTACGCA GACAGCAAAA CAAATCAAAT TTCTACAACA CAGAAAAATC     2610

AACAGAAAGA GATGGACCGA AAAGGATTAC TTGGGTATTA TTTCAAAGGA AAAGATTTTA     2670

GTAATCTTAC TATGTTTGCA CCGACACGTG ATAGTACTCT TATTTATGAT CAACAAACAG     2730

CAAATAAACT ATTAGATAAA AAACAACAAG AATATCAGTC TATTCGTTGG ATTGGTTTGA     2790

TTCAGAGTAA AGAAACGGGA GATTTCACAT TTAACTTATC TGAGGATGAA CAGGCAATTA     2850

TAGAAATCAA TGGGAAAATT ATTTCTAATA AAGGGAAAGA AAAGCAAGTT GTCCATTTAG     2910

AAAAAGGAAA ATTAGTTCCA ATCAAAATAG AGTATCAATC AGATACAAAA TTTAATATTG     2970

ACAGTAAAAC ATTTAAAGAA CTTAAATTAT TTAAAATAGA TAGTCAAAAC CAACCCCAGC     3030

AAGTCCAGCA AGATGAACTG AGAAATCCTG AATTTAACAA GAAAGAATCA CAGGAATTCT     3090

TAGCGAAACC ATCGAAAATA AATCTTTTCA CTCAAMAAAT GAAAAGGGAA ATTGATGAAG     3150

ACACGGATAC GGATGGGGAC TCTATTCCTG ACCTTTGGGA AGAAAATGGG TATACGATTC     3210

AMAATAGAAT CGCTGTAAAG TGGGACGATT CTCTAGCAAG TAAAGGGTAT ACGAAATTTG     3270

TTTCAAATCC ACTAGAAAGT CACACAGTTG GTGATCCTTA TACAGATTAT GAAAAGGCAG     3330

CAAGAGATCT AGATTTGTCA AATGCAAAGG AAACGTTTAA CCCATTGGTA GCTGCTTTTC     3390

CAAGTGTGAA TGTTAGTATG GAAAAGGTGA TATTATCACC AAATGAAAAT TTATCCAATA     3450

GTGTAGAGTC TCATTCATCC ACGAATTGGT CTTATACAAA TACAGAAGGT GCTTCTGTTG     3510

AAGCGGGGAT TGGACCAAAA GGTATTTCGT TCGGAGTTAG CGTAAACTAT CAACACTCTG     3570

AAACAGTTGC ACAAGAATGG GGAACATCTA CAGGAAATAC TTCGCAATTC AATACGGCTT     3630

CAGCGGGATA TTTAAATGCA AATGTTCGAT ATAACAATGT AGGAACTGGT GCCATCTACG     3690

ATGTAAAACC TACAACAAGT TTTGTATTAA ATAACGATAC TATCGCAACT ATTACGGCGA     3750

AATCTAATTC TACAGCCTTA AATATATCTC CTGGAGAAAG TTACCCGAAA AAAGGACAAA 3810
ATGGAATCGC AATAACATCA ATGGATGATT TTAATTCCCA TCCGATTACA TTAAATAAAA 3870
AACAAGTAGA TAATCTGCTA AATAATAAAC CTATGATGTT GGAAACAAAC CAAACAGATG 3930
GTGTTTATAA GATAAAAGAT ACACATGGAA ATATAGTAAC TGGCGGAGAA TGGAATGGTG 3990
TCATACAACA AATCAAGGCT AAAACAGCGT CTATTATTGT GGATGATGGG GAACGTGTAG 4050
CAGAAAAACG TGTAGCGGCA AAAGATTATG AAAATCCAGA AGATAAAACA CCGTCTTTAA 4110
CTTTAAAAGA TGCCCTGAAG CTTTCATATC CAGATGAAAT AAAAGAAATA GAGGGATTAT 4170
TATATTATAA AAACAAACCG ATATACGAAT CGAGCGTTAT GACTTACTTA GATGAAAATA 4230
CAGCAAAAGA AGTGACCAAA CAATTAAATG ATACCACTGG GAAATTTAAA GATGTAAGTC 4290
ATTTATATGA TGTAAAACTG ACTCCAAAAA TGAATGTTAC AATCAAATTG TCTATACTTT 4350
ATGATAATGC TGAGTCTAAT GATAACTCAA TTGGTAAATG GACAAACACA AATATTGTTT 4410
CAGGTGGAAA TAACGGAAAA AAACAATATT CTTCTAATAA TCCGGATGCT AATTTGACAT 4470
TAAATACAGA TGCTCAAGAA AAATTAAATA AAAATCGTGA CTATTATATA AGTTTATATA 4530
TGAAGTCAGA AAAAAACACA CAATGTGAGA TTACTATAGA TGGGGAGATT TATCCGATCA 4590
CTACAAAAAC AGTGAATGTG AATAAAGACA ATTACAAAAG ATTAGATATT ATAGCTCATA 4650
ATATAAAAAG TAATCCAATT TCTTCACTTC ATATTAAAAC GAATGATGAA ATAACTTTAT 4710
TTTGGGATGA TATTTCTATA ACAGATGTAG CATCAATAAA ACCGGAAAAT TTAACAGATT 4770
CAGAAATTAA ACAGATTTAT AGTAGGTATG GTATTAAGTT AGAAGATGGA ATCCTTATTG 4830
ATAAAAAAGG TGGGATTCAT TATGGTGAAT TTATTAATGA AGCTAGTTTT AATATTGAAC 4890
CATTGCCAAA TTATGTGACC AAATATGAAG TTACTTATAG TAGTGAGTTA GGACCAAACG 4950
TGAGTGACAC ACTTGAAAGT GATAAAATTT ACAAGGATGG GACAATTAAA TTTGATTTTA 5010
CCAAATATAG TAAAAATGAA CAAGGATTAT TTTATGACAG TGGATTAAAT TGGGACTTTA 5070
AAATTAATGC TATTACTTAT GATGGTAAAG AGATGAATGT TTTTCATAGA TATAATAAAT 5130
AGTTATTATA TCTATGAAGC TGGTGCTAAA GATAGTGTAA AAGTTAATAT ACTGTAGGAT 5190
TGTAATAAAA GTAATGGAAT TGATATCGTA CTTTGGAGTG GGGGATACTT TGTAAATAGT 5250
TCTATCAGAA ACATTAGACT AAGAAAAGTT ACTACCCCCA CTTGAAAATG AAGATTCAAC 5310
TGATTACAAA CAACCTGTTA AATATTATAA GGTTTTAACA AAATATTAAA CTCTTTATGT 5370
TAATACTGTA ATATAAAGAG TTTAATTGTA TTCAAATGAA GCTTTCCCAC AAAATTAGAC 5430
TGATTATCTA ATGAAATAAT CAGTCTAATT TTGTAGAACA GGTCTGGTAT TATTGTACGT 5490
GGTCACTAAA AGATATCTAA TATTATTGGG CAAGGCGTTC CATGATTGAA TCCTCGAATG 5550

TCTTGCCCTT TTCATTTATT TAAGAAGGAT TGTGGAGAAA TTATGGTTTA GATAATGAAG 5610

AAAGACTTCA CTTCTAATTT TTGATGTTAA ATAAATCAAA ATTTGGCGAT TCACATTGTT 5670

TAATCCACTG ATAAAACATA CTGGAGTGTT CTTAAAAAAT CAGCTTTTTT CTTTATAAAA 5730

TTTTGCTTAG CGTACGAAAT TCGTGTTTTG TTGGTGGGAC CCCATGCCCA TCAACTTAAG 5790

AGTAAATTAG TAATGAACTT TCGTTCATCT GGATTAAAAT AACCTCAAAT TAGGACATGT 5850

TTTTAAAAAT AAGCAGACCA AATAAGCCTA GAATAGGTAT CATTTTTAAA AATTATGCTG 5910

CTTTCTTTTG TTTTCCAAAT CCATTATACT CATAAGCAAC ACCCATAATG TCAAAGACTG 5970

TTTTTGTCTC ATATCGATAA GCTTGATATC GAATTCCTGC AGCCCGGGGG ATCCACTAGT 6030

TCTAGAGCGG CCGCCACCGC GGTGGAGCTC CAGCTTTTGT TCCCTTTAGT GAGGGTTAAG 6090

TTCGAGCTTG TCGTGG 6106

(2) INFORMATION FOR SEQ ID NO: 2:

(i) SEQUENCE CHARACTERISTICS:
(A) LENGTH: 243 amino acids
(B) TYPE: amino acid
(D) TOPOLOGY: linear

(ii) MOLECULE TYPE: protein

(xi) SEQUENCE DESCRIPTION: SEQ ID NO: 2:

Met Lys Arg Met Glu Gly Lys Leu Phe Met Val Ser Lys Lys Leu Gln
1               5                   10                  15

Val Val Thr Lys Thr Val Leu Leu Ser Thr Val Phe Ser Ile Ser Leu
            20                  25                  30

Leu Asn Asn Glu Val Ile Lys Ala Glu Gln Leu Asn Ile Asn Ser Gln
        35                  40                  45

Ser Lys Tyr Thr Asn Leu Gln Asn Leu Lys Ile Thr Asp Lys Val Glu
    50                  55                  60

Asp Phe Lys Glu Asp Lys Glu Lys Ala Lys Glu Trp Gly Lys Glu Lys
65                  70                  75                  80

Glu Lys Glu Trp Lys Leu Thr Ala Thr Glu Lys Gly Lys Met Asn Asn
                85                  90                  95

Phe Leu Asp Asn Lys Asn Asp Ile Xaa Thr Asn Tyr Lys Glu Ile Thr
            100                 105                 110

Phe Ser Met Ala Gly Ser Phe Glu Asp Glu Ile Lys Asp Leu Lys Glu
        115                 120                 125

Ile Asp Lys Met Phe Asp Lys Thr Asn Leu Ser Asn Ser Ile Ile Thr
    130                 135                 140

Tyr Lys Asn Val Glu Pro Thr Thr Ile Gly Phe Asn Lys Ser Leu Thr
145                 150                 155                 160

Glu Gly Asn Thr Ile Asn Ser Asp Ala Met Ala Gln Phe Lys Glu Gln
                165                 170                 175

Phe Leu Asp Arg Asp Ile Lys Phe Asp Ser Tyr Leu Asp Thr His Leu
            180                 185                 190

Thr Ala Gln Gln Val Ser Ser Lys Glu Arg Val Ile Leu Lys Val Thr
        195                 200                 205

Val Pro Ser Gly Lys Gly Ser Thr Thr Pro Thr Lys Ala Gly Val Ile
    210                 215                 220

Leu Asn Asn Ser Glu Tyr Lys Met Leu Ile Asp Asn Gly Tyr Met Val
225                 230                 235                 240

His Val Asp

(2) INFORMATION FOR SEQ ID NO: 3:

(i) SEQUENCE CHARACTERISTICS:
(A) LENGTH: 182 amino acids
(B) TYPE: amino acid
(D) TOPOLOGY: linear

(ii) MOLECULE TYPE: protein

(xi) SEQUENCE DESCRIPTION: SEQ ID NO: 3:

Met Lys Asn Tyr Glu Glu Trp Ala Lys Asp Leu Thr Asp Ser Gln Arg
  1               5                  10                  15

Glu Ala Leu Asp Gly Tyr Ala Arg Gln Asp Tyr Lys Glu Ile Asn Asn
            20                  25                  30

Tyr Leu Arg Asn Gln Gly Gly Ser Gly Asn Glu Lys Leu Asp Ala Gln
        35                  40                  45

Ile Lys Asn Ile Ser Asp Ala Leu Gly Lys Lys Pro Ile Pro Glu Asn
    50                  55                  60

Ile Thr Val Tyr Arg Trp Cys Gly Met Pro Glu Phe Gly Tyr Gln Ile
65                  70                  75                  80

Ser Asp Pro Leu Pro Ser Leu Lys Asp Phe Glu Glu Gln Phe Leu Asn
                85                  90                  95

Thr Ile Lys Glu Asp Lys Gly Tyr Met Ser Thr Ser Leu Ser Ser Glu
            100                 105                 110

Arg Leu Ala Ala Phe Gly Ser Arg Lys Ile Ile Leu Arg Leu Gln Val
        115                 120                 125

Pro Lys Gly Ser Thr Gly Ala Tyr Leu Ser Ala Ile Gly Gly Phe Ala
    130                 135                 140

Ser Glu Lys Glu Ile Leu Leu Asp Lys Asp Ser Lys Tyr His Ile Asp
145                 150                 155                 160

Lys Val Thr Glu Val Ile Ile Lys Gly Val Lys Arg Tyr Val Val Asp
                165                 170                 175

Ala Thr Leu Leu Thr Asn
            180

(2) INFORMATION FOR SEQ ID NO: 4:

(i) SEQUENCE CHARACTERISTICS:
(A) LENGTH: 2655 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: CDS
(B) LOCATION: 1..2652
(C) IDENTIFICATION METHOD: experimental
(D) OTHER INFORMATION: /product= "100 kDa protein VIP-1"
/evidence= EXPERIMENTAL

(xi) SEQUENCE DESCRIPTION: SEQ ID NO: 4:

ATG AAA AAT ATG AAG AAA AAG TTA GCA AGT GTT GTA ACG TGT ACG TTA      48
Met Lys Asn Met Lys Lys Lys Leu Ala Ser Val Val Thr Cys Thr Leu
  1               5                  10                  15

TTA GCT CCT ATG TTT TTG AAT GGA AAT GTG AAT GCT GTT TAC GCA GAC      96
Leu Ala Pro Met Phe Leu Asn Gly Asn Val Asn Ala Val Tyr Ala Asp
             20                  25                  30

AGC AAA ACA AAT CAA ATT TCT ACA ACA CAG AAA AAT CAA CAG AAA GAG     144
Ser Lys Thr Asn Gln Ile Ser Thr Thr Gln Lys Asn Gln Gln Lys Glu
         35                  40                  45

ATG GAC CGA AAA GGA TTA CTT GGG TAT TAT TTC AAA GGA AAA GAT TTT     192
Met Asp Arg Lys Gly Leu Leu Gly Tyr Tyr Phe Lys Gly Lys Asp Phe
     50                  55                  60

AGT AAT CTT ACT ATG TTT GCA CCG ACA CGT GAT AGT ACT CTT ATT TAT     240
Ser Asn Leu Thr Met Phe Ala Pro Thr Arg Asp Ser Thr Leu Ile Tyr
 65                  70                  75                  80

GAT CAA CAA ACA GCA AAT AAA CTA TTA GAT AAA AAA CAA CAA GAA TAT     288
Asp Gln Gln Thr Ala Asn Lys Leu Leu Asp Lys Lys Gln Gln Glu Tyr

                85                  90                  95
CAG TCT ATT CGT TGG ATT GGT TTG ATT CAG AGT AAA GAA ACG GGA GAT 336
Gln Ser Ile Arg Trp Ile Gly Leu Ile Gln Ser Lys Glu Thr Gly Asp
            100                 105                 110
TTC ACA TTT AAC TTA TCT GAG GAT GAA CAG GCA ATT ATA GAA ATC AAT 384
Phe Thr Phe Asn Leu Ser Glu Asp Glu Gln Ala Ile Ile Glu Ile Asn
        115                 120                 125
GGG AAA ATT ATT TCT AAT AAA GGG AAA GAA AAG CAA GTT GTC CAT TTA 432
Gly Lys Ile Ile Ser Asn Lys Gly Lys Glu Lys Gln Val Val His Leu
    130                 135                 140
GAA AAA GGA AAA TTA GTT CCA ATC AAA ATA GAG TAT CAA TCA GAT ACA 480
Glu Lys Gly Lys Leu Val Pro Ile Lys Ile Glu Tyr Gln Ser Asp Thr
145                 150                 155                 160
AAA TTT AAT ATT GAC AGT AAA ACA TTT AAA GAA CTT AAA TTA TTT AAA 528
Lys Phe Asn Ile Asp Ser Lys Thr Phe Lys Glu Leu Lys Leu Phe Lys
                165                 170                 175
ATA GAT AGT CAA AAC CAA CCC CAG CAA GTC CAG CAA GAT GAA CTG AGA 576
Ile Asp Ser Gln Asn Gln Pro Gln Gln Val Gln Gln Asp Glu Leu Arg
            180                 185                 190
AAT CCT GAA TTT AAC AAG AAA GAA TCA CAG GAA TTC TTA GCG AAA CCA 624
Asn Pro Glu Phe Asn Lys Lys Glu Ser Gln Glu Phe Leu Ala Lys Pro
        195                 200                 205
TCG AAA ATA AAT CTT TTC ACT CAA MAA ATG AAA AGG GAA ATT GAT GAA 672
Ser Lys Ile Asn Leu Phe Thr Gln Xaa Met Lys Arg Glu Ile Asp Glu
    210                 215                 220
GAC ACG GAT ACG GAT GGG GAC TCT ATT CCT GAC CTT TGG GAA GAA AAT 720
Asp Thr Asp Thr Asp Gly Asp Ser Ile Pro Asp Leu Trp Glu Glu Asn
225                 230                 235                 240
GGG TAT ACG ATT CAM AAT AGA ATC GCT GTA AAG TGG GAC GAT TCT CTA 768
Gly Tyr Thr Ile Xaa Asn Arg Ile Ala Val Lys Trp Asp Asp Ser Leu
                245                 250                 255
GCA AGT AAA GGG TAT ACG AAA TTT GTT TCA AAT CCA CTA GAA AGT CAC 816
Ala Ser Lys Gly Tyr Thr Lys Phe Val Ser Asn Pro Leu Glu Ser His
            260                 265                 270
ACA GTT GGT GAT CCT TAT ACA GAT TAT GAA AAG GCA GCA AGA GAT CTA 864
Thr Val Gly Asp Pro Tyr Thr Asp Tyr Glu Lys Ala Ala Arg Asp Leu
        275                 280                 285
GAT TTG TCA AAT GCA AAG GAA ACG TTT AAC CCA TTG GTA GCT GCT TTT 912
Asp Leu Ser Asn Ala Lys Glu Thr Phe Asn Pro Leu Val Ala Ala Phe
    290                 295                 300
CCA AGT GTG AAT GTT AGT ATG GAA AAG GTG ATA TTA TCA CCA AAT GAA 960
Pro Ser Val Asn Val Ser Met Glu Lys Val Ile Leu Ser Pro Asn Glu
305                 310                 315                 320
AAT TTA TCC AAT AGT GTA GAG TCT CAT TCA TCC ACG AAT TGG TCT TAT 1008
Asn Leu Ser Asn Ser Val Glu Ser His Ser Ser Thr Asn Trp Ser Tyr

                325                 330                 335

ACA AAT ACA GAA GGT GCT TCT GTT GAA GCG GGG ATT GGA CCA AAA GGT 1056
Thr Asn Thr Glu Gly Ala Ser Val Glu Ala Gly Ile Gly Pro Lys Gly
            340                 345                 350

ATT TCG TTC GGA GTT AGC GTA AAC TAT CAA CAC TCT GAA ACA GTT GCA 1104
Ile Ser Phe Gly Val Ser Val Asn Tyr Gln His Ser Glu Thr Val Ala
        355                 360                 365

CAA GAA TGG GGA ACA TCT ACA GGA AAT ACT TCG CAA TTC AAT ACG GCT 1152
Gln Glu Trp Gly Thr Ser Thr Gly Asn Thr Ser Gln Phe Asn Thr Ala
    370                 375                 380

TCA GCG GGA TAT TTA AAT GCA AAT GTT CGA TAT AAC AAT GTA GGA ACT 1200
Ser Ala Gly Tyr Leu Asn Ala Asn Val Arg Tyr Asn Asn Val Gly Thr
385                 390                 395                 400

GGT GCC ATC TAC GAT GTA AAA CCT ACA ACA AGT TTT GTA TTA AAT AAC 1248
Gly Ala Ile Tyr Asp Val Lys Pro Thr Thr Ser Phe Val Leu Asn Asn
                405                 410                 415

GAT ACT ATC GCA ACT ATT ACG GCG AAA TCT AAT TCT ACA GCC TTA AAT 1296
Asp Thr Ile Ala Thr Ile Thr Ala Lys Ser Asn Ser Thr Ala Leu Asn
            420                 425                 430

ATA TCT CCT GGA GAA AGT TAC CCG AAA AAA GGA CAA AAT GGA ATC GCA 1344
Ile Ser Pro Gly Glu Ser Tyr Pro Lys Lys Gly Gln Asn Gly Ile Ala
        435                 440                 445

ATA ACA TCA ATG GAT GAT TTT AAT TCC CAT CCG ATT ACA TTA AAT AAA 1392
Ile Thr Ser Met Asp Asp Phe Asn Ser His Pro Ile Thr Leu Asn Lys
    450                 455                 460

AAA CAA GTA GAT AAT CTG CTA AAT AAT AAA CCT ATG ATG TTG GAA ACA 1440
Lys Gln Val Asp Asn Leu Leu Asn Asn Lys Pro Met Met Leu Glu Thr
465                 470                 475                 480

AAC CAA ACA GAT GGT GTT TAT AAG ATA AAA GAT ACA CAT GGA AAT ATA 1488
Asn Gln Thr Asp Gly Val Tyr Lys Ile Lys Asp Thr His Gly Asn Ile
                485                 490                 495

GTA ACT GGC GGA GAA TGG AAT GGT GTC ATA CAA CAA ATC AAG GCT AAA 1536
Val Thr Gly Gly Glu Trp Asn Gly Val Ile Gln Gln Ile Lys Ala Lys
            500                 505                 510

ACA GCG TCT ATT ATT GTG GAT GAT GGG GAA CGT GTA GCA GAA AAA CGT 1584
Thr Ala Ser Ile Ile Val Asp Asp Gly Glu Arg Val Ala Glu Lys Arg
        515                 520                 525

GTA GCG GCA AAA GAT TAT GAA AAT CCA GAA GAT AAA ACA CCG TCT TTA 1632
Val Ala Ala Lys Asp Tyr Glu Asn Pro Glu Asp Lys Thr Pro Ser Leu
    530                 535                 540

ACT TTA AAA GAT GCC CTG AAG CTT TCA TAT CCA GAT GAA ATA AAA GAA 1680
Thr Leu Lys Asp Ala Leu Lys Leu Ser Tyr Pro Asp Glu Ile Lys Glu
545                 550                 555                 560

ATA GAG GGA TTA TTA TAT TAT AAA AAC AAA CCG ATA TAC GAA TCG AGC 1728
Ile Glu Gly Leu Leu Tyr Tyr Lys Asn Lys Pro Ile Tyr Glu Ser Ser

                565                 570                 575

GTT ATG ACT TAC TTA GAT GAA AAT ACA GCA AAA GAA GTG ACC AAA CAA 1776
Val Met Thr Tyr Leu Asp Glu Asn Thr Ala Lys Glu Val Thr Lys Gln
            580                 585                 590

TTA AAT GAT ACC ACT GGG AAA TTT AAA GAT GTA AGT CAT TTA TAT GAT 1824
Leu Asn Asp Thr Thr Gly Lys Phe Lys Asp Val Ser His Leu Tyr Asp
        595                 600                 605

GTA AAA CTG ACT CCA AAA ATG AAT GTT ACA ATC AAA TTG TCT ATA CTT 1872
Val Lys Leu Thr Pro Lys Met Asn Val Thr Ile Lys Leu Ser Ile Leu
    610                 615                 620

TAT GAT AAT GCT GAG TCT AAT GAT AAC TCA ATT GGT AAA TGG ACA AAC 1920
Tyr Asp Asn Ala Glu Ser Asn Asp Asn Ser Ile Gly Lys Trp Thr Asn
625                 630                 635                 640

ACA AAT ATT GTT TCA GGT GGA AAT AAC GGA AAA AAA CAA TAT TCT TCT 1968
Thr Asn Ile Val Ser Gly Gly Asn Asn Gly Lys Lys Gln Tyr Ser Ser
                645                 650                 655

AAT AAT CCG GAT GCT AAT TTG ACA TTA AAT ACA GAT GCT CAA GAA AAA 2016
Asn Asn Pro Asp Ala Asn Leu Thr Leu Asn Thr Asp Ala Gln Glu Lys
            660                 665                 670

TTA AAT AAA AAT CGT GAC TAT TAT ATA AGT TTA TAT ATG AAG TCA GAA 2064
Leu Asn Lys Asn Arg Asp Tyr Tyr Ile Ser Leu Tyr Met Lys Ser Glu
        675                 680                 685

AAA AAC ACA CAA TGT GAG ATT ACT ATA GAT GGG GAG ATT TAT CCG ATC 2112
Lys Asn Thr Gln Cys Glu Ile Thr Ile Asp Gly Glu Ile Tyr Pro Ile
    690                 695                 700

ACT ACA AAA ACA GTG AAT GTG AAT AAA GAC AAT TAC AAA AGA TTA GAT 2160
Thr Thr Lys Thr Val Asn Val Asn Lys Asp Asn Tyr Lys Arg Leu Asp
705                 710                 715                 720

ATT ATA GCT CAT AAT ATA AAA AGT AAT CCA ATT TCT TCA CTT CAT ATT 2208
Ile Ile Ala His Asn Ile Lys Ser Asn Pro Ile Ser Ser Leu His Ile
                725                 730                 735

AAA ACG AAT GAT GAA ATA ACT TTA TTT TGG GAT GAT ATT TCT ATA ACA 2256
Lys Thr Asn Asp Glu Ile Thr Leu Phe Trp Asp Asp Ile Ser Ile Thr
            740                 745                 750

GAT GTA GCA TCA ATA AAA CCG GAA AAT TTA ACA GAT TCA GAA ATT AAA 2304
Asp Val Ala Ser Ile Lys Pro Glu Asn Leu Thr Asp Ser Glu Ile Lys
        755                 760                 765

CAG ATT TAT AGT AGG TAT GGT ATT AAG TTA GAA GAT GGA ATC CTT ATT 2352
Gln Ile Tyr Ser Arg Tyr Gly Ile Lys Leu Glu Asp Gly Ile Leu Ile
    770                 775                 780

GAT AAA AAA GGT GGG ATT CAT TAT GGT GAA TTT ATT AAT GAA GCT AGT 2400
Asp Lys Lys Gly Gly Ile His Tyr Gly Glu Phe Ile Asn Glu Ala Ser
785                 790                 795                 800

TTT AAT ATT GAA CCA TTG CCA AAT TAT GTG ACC AAA TAT GAA GTT ACT 2448
Phe Asn Ile Glu Pro Leu Pro Asn Tyr Val Thr Lys Tyr Glu Val Thr

                805                 810                 815

TAT AGT AGT GAG TTA GGA CCA AAC GTG AGT GAC ACA CTT GAA AGT GAT 2496
Tyr Ser Ser Glu Leu Gly Pro Asn Val Ser Asp Thr Leu Glu Ser Asp
            820                 825                 830

AAA ATT TAC AAG GAT GGG ACA ATT AAA TTT GAT TTT ACC AAA TAT AGT 2544
Lys Ile Tyr Lys Asp Gly Thr Ile Lys Phe Asp Phe Thr Lys Tyr Ser
        835                 840                 845

AAA AAT GAA CAA GGA TTA TTT TAT GAC AGT GGA TTA AAT TGG GAC TTT 2592
Lys Asn Glu Gln Gly Leu Phe Tyr Asp Ser Gly Leu Asn Trp Asp Phe
    850                 855                 860

AAA ATT AAT GCT ATT ACT TAT GAT GGT AAA GAG ATG AAT GTT TTT CAT 2640
Lys Ile Asn Ala Ile Thr Tyr Asp Gly Lys Glu Met Asn Val Phe His
865                 870                 875                 880

AGA TAT AAT AAA TAG 2655
Arg Tyr Asn Lys

(2) INFORMATION FOR SEQ ID NO: 5:

(i) SEQUENCE CHARACTERISTICS:
(A) LENGTH: 884 amino acids
(B) TYPE: amino acid
(D) TOPOLOGY: linear

(ii) MOLECULE TYPE: protein

(xi) SEQUENCE DESCRIPTION: SEQ ID NO: 5:

Met Lys Asn Met Lys Lys Lys Leu Ala Ser Val Val Thr Cys Thr Leu
1               5                   10                  15

Leu Ala Pro Met Phe Leu Asn Gly Asn Val Asn Ala Val Tyr Ala Asp
            20                  25                  30

Ser Lys Thr Asn Gln Ile Ser Thr Thr Gln Lys Asn Gln Gln Lys Glu
        35                  40                  45

Met Asp Arg Lys Gly Leu Leu Gly Tyr Tyr Phe Lys Gly Lys Asp Phe
    50                  55                  60

Ser Asn Leu Thr Met Phe Ala Pro Thr Arg Asp Ser Thr Leu Ile Tyr
65                  70                  75                  80

Asp Gln Gln Thr Ala Asn Lys Leu Leu Asp Lys Lys Gln Gln Glu Tyr
                85                  90                  95

Gln Ser Ile Arg Trp Ile Gly Leu Ile Gln Ser Lys Glu Thr Gly Asp
            100                 105                 110

Phe Thr Phe Asn Leu Ser Glu Asp Glu Gln Ala Ile Ile Glu Ile Asn
        115                 120                 125

Gly Lys Ile Ile Ser Asn Lys Gly Lys Glu Lys Gln Val Val His Leu
    130                 135                 140

Glu Lys Gly Lys Leu Val Pro Ile Lys Ile Glu Tyr Gln Ser Asp Thr
145                 150                 155                 160

Lys Phe Asn Ile Asp Ser Lys Thr Phe Lys Glu Leu Lys Leu Phe Lys
                165                 170                 175

Ile Asp Ser Gln Asn Gln Pro Gln Gln Val Gln Gln Asp Glu Leu Arg
            180                 185                 190

Asn Pro Glu Phe Asn Lys Lys Glu Ser Gln Glu Phe Leu Ala Lys Pro
        195                 200                 205

Ser Lys Ile Asn Leu Phe Thr Gln Xaa Met Lys Arg Glu Ile Asp Glu
    210                 215                 220

Asp Thr Asp Thr Asp Gly Asp Ser Ile Pro Asp Leu Trp Glu Glu Asn
225                 230                 235                 240

Gly Tyr Thr Ile Xaa Asn Arg Ile Ala Val Lys Trp Asp Asp Ser Leu
                245                 250                 255

Ala Ser Lys Gly Tyr Thr Lys Phe Val Ser Asn Pro Leu Glu Ser His
            260                 265                 270

Thr Val Gly Asp Pro Tyr Thr Asp Tyr Glu Lys Ala Ala Arg Asp Leu
        275                 280                 285

Asp Leu Ser Asn Ala Lys Glu Thr Phe Asn Pro Leu Val Ala Ala Phe
    290                 295                 300

Pro Ser Val Asn Val Ser Met Glu Lys Val Ile Leu Ser Pro Asn Glu
305                 310                 315                 320

Asn Leu Ser Asn Ser Val Glu Ser His Ser Ser Thr Asn Trp Ser Tyr
                325                 330                 335

Thr Asn Thr Glu Gly Ala Ser Val Glu Ala Gly Ile Gly Pro Lys Gly
            340                 345                 350

Ile Ser Phe Gly Val Ser Val Asn Tyr Gln His Ser Glu Thr Val Ala
        355                 360                 365

Gln Glu Trp Gly Thr Ser Thr Gly Asn Thr Ser Gln Phe Asn Thr Ala
    370                 375                 380

Ser Ala Gly Tyr Leu Asn Ala Asn Val Arg Tyr Asn Asn Val Gly Thr
385                 390                 395                 400

Gly Ala Ile Tyr Asp Val Lys Pro Thr Thr Ser Phe Val Leu Asn Asn
                405                 410                 415

Asp Thr Ile Ala Thr Ile Thr Ala Lys Ser Asn Ser Thr Ala Leu Asn
            420                 425                 430

Ile Ser Pro Gly Glu Ser Tyr Pro Lys Lys Gly Gln Asn Gly Ile Ala
        435                 440                 445

Ile Thr Ser Met Asp Asp Phe Asn Ser His Pro Ile Thr Leu Asn Lys
    450                 455                 460

Lys Gln Val Asp Asn Leu Leu Asn Asn Lys Pro Met Met Leu Glu Thr
465                 470                 475                 480

Asn Gln Thr Asp Gly Val Tyr Lys Ile Lys Asp Thr His Gly Asn Ile
                485                 490                 495

Val Thr Gly Gly Glu Trp Asn Gly Val Ile Gln Gln Ile Lys Ala Lys
            500                 505                 510

Thr Ala Ser Ile Ile Val Asp Asp Gly Glu Arg Val Ala Glu Lys Arg
        515                 520                 525

Val Ala Ala Lys Asp Tyr Glu Asn Pro Glu Asp Lys Thr Pro Ser Leu
    530                 535                 540

Thr Leu Lys Asp Ala Leu Lys Leu Ser Tyr Pro Asp Glu Ile Lys Glu
545                 550                 555                 560

Ile Glu Gly Leu Leu Tyr Tyr Lys Asn Lys Pro Ile Tyr Glu Ser Ser
                565                 570                 575

Val Met Thr Tyr Leu Asp Glu Asn Thr Ala Lys Glu Val Thr Lys Gln
            580                 585                 590

Leu Asn Asp Thr Thr Gly Lys Phe Lys Asp Val Ser His Leu Tyr Asp
        595                 600                 605

Val Lys Leu Thr Pro Lys Met Asn Val Thr Ile Lys Leu Ser Ile Leu
    610                 615                 620

Tyr Asp Asn Ala Glu Ser Asn Asp Asn Ser Ile Gly Lys Trp Thr Asn
625                 630                 635                 640

Thr Asn Ile Val Ser Gly Gly Asn Asn Gly Lys Lys Gln Tyr Ser Ser
                645                 650                 655

Asn Asn Pro Asp Ala Asn Leu Thr Leu Asn Thr Asp Ala Gln Glu Lys
            660                 665                 670

Leu Asn Lys Asn Arg Asp Tyr Tyr Ile Ser Leu Tyr Met Lys Ser Glu
        675                 680                 685

Lys Asn Thr Gln Cys Glu Ile Thr Ile Asp Gly Glu Ile Tyr Pro Ile
    690                 695                 700

Thr Thr Lys Thr Val Asn Val Asn Lys Asp Asn Tyr Lys Arg Leu Asp
705                 710                 715                 720

Ile Ile Ala His Asn Ile Lys Ser Asn Pro Ile Ser Ser Leu His Ile
                725                 730                 735

Lys Thr Asn Asp Glu Ile Thr Leu Phe Trp Asp Asp Ile Ser Ile Thr
            740                 745                 750

Asp Val Ala Ser Ile Lys Pro Glu Asn Leu Thr Asp Ser Glu Ile Lys
        755                 760                 765

Gln Ile Tyr Ser Arg Tyr Gly Ile Lys Leu Glu Asp Gly Ile Leu Ile
    770                 775                 780

Asp Lys Lys Gly Gly Ile His Tyr Gly Glu Phe Ile Asn Glu Ala Ser
785                 790                 795                 800

Phe Asn Ile Glu Pro Leu Pro Asn Tyr Val Thr Lys Tyr Glu Val Thr
                805                 810                 815

Tyr Ser Ser Glu Leu Gly Pro Asn Val Ser Asp Thr Leu Glu Ser Asp
            820                 825                 830

Lys Ile Tyr Lys Asp Gly Thr Ile Lys Phe Asp Phe Thr Lys Tyr Ser
        835                 840                 845

Lys Asn Glu Gln Gly Leu Phe Tyr Asp Ser Gly Leu Asn Trp Asp Phe
    850                 855                 860

Lys Ile Asn Ala Ile Thr Tyr Asp Gly Lys Glu Met Asn Val Phe His
865                 870                 875                 880

Arg Tyr Asn Lys

(2) INFORMATION FOR SEQ ID NO: 6:

(i) SEQUENCE CHARACTERISTICS:
(A) LENGTH: 2004 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: CDS
(B) LOCATION: 1..2001
(C) IDENTIFICATION METHOD: experimental
(D) OTHER INFORMATION: /product= "80 kDa protein VIP-1"
/evidence= EXPERIMENTAL

(xi) SEQUENCE DESCRIPTION: SEQ ID NO: 6:

ATG AAA AGG GAA ATT GAT GAA GAC ACG GAT ACG GAT GGG GAC TCT ATT      48
Met Lys Arg Glu Ile Asp Glu Asp Thr Asp Thr Asp Gly Asp Ser Ile
  1               5                  10                  15

CCT GAC CTT TGG GAA GAA AAT GGG TAT ACG ATT CAM AAT AGA ATC GCT      96
Pro Asp Leu Trp Glu Glu Asn Gly Tyr Thr Ile Xaa Asn Arg Ile Ala
             20                  25                  30

GTA AAG TGG GAC GAT TCT CTA GCA AGT AAA GGG TAT ACG AAA TTT GTT     144

Val Lys Trp Asp Asp Ser Leu Ala Ser Lys Gly Tyr Thr Lys Phe Val
        35                  40                  45

TCA AAT CCA CTA GAA AGT CAC ACA GTT GGT GAT CCT TAT ACA GAT TAT 192
Ser Asn Pro Leu Glu Ser His Thr Val Gly Asp Pro Tyr Thr Asp Tyr
    50                  55                  60

GAA AAG GCA GCA AGA GAT CTA GAT TTG TCA AAT GCA AAG GAA ACG TTT 240
Glu Lys Ala Ala Arg Asp Leu Asp Leu Ser Asn Ala Lys Glu Thr Phe
65                  70                  75                  80

AAC CCA TTG GTA GCT GCT TTT CCA AGT GTG AAT GTT AGT ATG GAA AAG 288
Asn Pro Leu Val Ala Ala Phe Pro Ser Val Asn Val Ser Met Glu Lys
                85                  90                  95

GTG ATA TTA TCA CCA AAT GAA AAT TTA TCC AAT AGT GTA GAG TCT CAT 336
Val Ile Leu Ser Pro Asn Glu Asn Leu Ser Asn Ser Val Glu Ser His
            100                 105                 110

TCA TCC ACG AAT TGG TCT TAT ACA AAT ACA GAA GGT GCT TCT GTT GAA 384
Ser Ser Thr Asn Trp Ser Tyr Thr Asn Thr Glu Gly Ala Ser Val Glu
        115                 120                 125

GCG GGG ATT GGA CCA AAA GGT ATT TCG TTC GGA GTT AGC GTA AAC TAT 432
Ala Gly Ile Gly Pro Lys Gly Ile Ser Phe Gly Val Ser Val Asn Tyr
    130                 135                 140

CAA CAC TCT GAA ACA GTT GCA CAA GAA TGG GGA ACA TCT ACA GGA AAT 480
Gln His Ser Glu Thr Val Ala Gln Glu Trp Gly Thr Ser Thr Gly Asn
145                 150                 155                 160

ACT TCG CAA TTC AAT ACG GCT TCA GCG GGA TAT TTA AAT GCA AAT GTT 528
Thr Ser Gln Phe Asn Thr Ala Ser Ala Gly Tyr Leu Asn Ala Asn Val
                165                 170                 175

CGA TAT AAC AAT GTA GGA ACT GGT GCC ATC TAC GAT GTA AAA CCT ACA 576
Arg Tyr Asn Asn Val Gly Thr Gly Ala Ile Tyr Asp Val Lys Pro Thr
            180                 185                 190

ACA AGT TTT GTA TTA AAT AAC GAT ACT ATC GCA ACT ATT ACG GCG AAA 624
Thr Ser Phe Val Leu Asn Asn Asp Thr Ile Ala Thr Ile Thr Ala Lys
        195                 200                 205

TCT AAT TCT ACA GCC TTA AAT ATA TCT CCT GGA GAA AGT TAC CCG AAA 672
Ser Asn Ser Thr Ala Leu Asn Ile Ser Pro Gly Glu Ser Tyr Pro Lys
    210                 215                 220

AAA GGA CAA AAT GGA ATC GCA ATA ACA TCA ATG GAT GAT TTT AAT TCC 720
Lys Gly Gln Asn Gly Ile Ala Ile Thr Ser Met Asp Asp Phe Asn Ser
225                 230                 235                 240

CAT CCG ATT ACA TTA AAT AAA AAA CAA GTA GAT AAT CTG CTA AAT AAT 768
His Pro Ile Thr Leu Asn Lys Lys Gln Val Asp Asn Leu Leu Asn Asn
                245                 250                 255

AAA CCT ATG ATG TTG GAA ACA AAC CAA ACA GAT GGT GTT TAT AAG ATA 816
Lys Pro Met Met Leu Glu Thr Asn Gln Thr Asp Gly Val Tyr Lys Ile
            260                 265                 270

AAA GAT ACA CAT GGA AAT ATA GTA ACT GGC GGA GAA TGG AAT GGT GTC 864

Lys Asp Thr His Gly Asn Ile Val Thr Gly Gly Glu Trp Asn Gly Val
        275                 280                 285

ATA CAA CAA ATC AAG GCT AAA ACA GCG TCT ATT ATT GTG GAT GAT GGG     912
Ile Gln Gln Ile Lys Ala Lys Thr Ala Ser Ile Ile Val Asp Asp Gly
    290                 295                 300

GAA CGT GTA GCA GAA AAA CGT GTA GCG GCA AAA GAT TAT GAA AAT CCA     960
Glu Arg Val Ala Glu Lys Arg Val Ala Ala Lys Asp Tyr Glu Asn Pro
305                 310                 315                 320

GAA GAT AAA ACA CCG TCT TTA ACT TTA AAA GAT GCC CTG AAG CTT TCA     1008
Glu Asp Lys Thr Pro Ser Leu Thr Leu Lys Asp Ala Leu Lys Leu Ser
                325                 330                 335

TAT CCA GAT GAA ATA AAA GAA ATA GAG GGA TTA TTA TAT TAT AAA AAC     1056
Tyr Pro Asp Glu Ile Lys Glu Ile Glu Gly Leu Leu Tyr Tyr Lys Asn
            340                 345                 350

AAA CCG ATA TAC GAA TCG AGC GTT ATG ACT TAC TTA GAT GAA AAT ACA     1104
Lys Pro Ile Tyr Glu Ser Ser Val Met Thr Tyr Leu Asp Glu Asn Thr
        355                 360                 365

GCA AAA GAA GTG ACC AAA CAA TTA AAT GAT ACC ACT GGG AAA TTT AAA     1152
Ala Lys Glu Val Thr Lys Gln Leu Asn Asp Thr Thr Gly Lys Phe Lys
    370                 375                 380

GAT GTA AGT CAT TTA TAT GAT GTA AAA CTG ACT CCA AAA ATG AAT GTT     1200
Asp Val Ser His Leu Tyr Asp Val Lys Leu Thr Pro Lys Met Asn Val
385                 390                 395                 400

ACA ATC AAA TTG TCT ATA CTT TAT GAT AAT GCT GAG TCT AAT GAT AAC     1248
Thr Ile Lys Leu Ser Ile Leu Tyr Asp Asn Ala Glu Ser Asn Asp Asn
                405                 410                 415

TCA ATT GGT AAA TGG ACA AAC ACA AAT ATT GTT TCA GGT GGA AAT AAC     1296
Ser Ile Gly Lys Trp Thr Asn Thr Asn Ile Val Ser Gly Gly Asn Asn
            420                 425                 430

GGA AAA AAA CAA TAT TCT TCT AAT AAT CCG GAT GCT AAT TTG ACA TTA     1344
Gly Lys Lys Gln Tyr Ser Ser Asn Asn Pro Asp Ala Asn Leu Thr Leu
        435                 440                 445

AAT ACA GAT GCT CAA GAA AAA TTA AAT AAA AAT CGT GAC TAT TAT ATA     1392
Asn Thr Asp Ala Gln Glu Lys Leu Asn Lys Asn Arg Asp Tyr Tyr Ile
    450                 455                 460

AGT TTA TAT ATG AAG TCA GAA AAA AAC ACA CAA TGT GAG ATT ACT ATA     1440
Ser Leu Tyr Met Lys Ser Glu Lys Asn Thr Gln Cys Glu Ile Thr Ile
465                 470                 475                 480

GAT GGG GAG ATT TAT CCG ATC ACT ACA AAA ACA GTG AAT GTG AAT AAA     1488
Asp Gly Glu Ile Tyr Pro Ile Thr Thr Lys Thr Val Asn Val Asn Lys
                485                 490                 495

GAC AAT TAC AAA AGA TTA GAT ATT ATA GCT CAT AAT ATA AAA AGT AAT     1536
Asp Asn Tyr Lys Arg Leu Asp Ile Ile Ala His Asn Ile Lys Ser Asn
            500                 505                 510

CCA ATT TCT TCA CTT CAT ATT AAA ACG AAT GAT GAA ATA ACT TTA TTT     1584

Pro Ile Ser Ser Leu His Ile Lys Thr Asn Asp Glu Ile Thr Leu Phe
        515                 520                 525

TGG GAT GAT ATT TCT ATA ACA GAT GTA GCA TCA ATA AAA CCG GAA AAT        1632
Trp Asp Asp Ile Ser Ile Thr Asp Val Ala Ser Ile Lys Pro Glu Asn
    530                 535                 540

TTA ACA GAT TCA GAA ATT AAA CAG ATT TAT AGT AGG TAT GGT ATT AAG        1680
Leu Thr Asp Ser Glu Ile Lys Gln Ile Tyr Ser Arg Tyr Gly Ile Lys
545                 550                 555                 560

TTA GAA GAT GGA ATC CTT ATT GAT AAA AAA GGT GGG ATT CAT TAT GGT        1728
Leu Glu Asp Gly Ile Leu Ile Asp Lys Lys Gly Gly Ile His Tyr Gly
                565                 570                 575

GAA TTT ATT AAT GAA GCT AGT TTT AAT ATT GAA CCA TTG CCA AAT TAT        1776
Glu Phe Ile Asn Glu Ala Ser Phe Asn Ile Glu Pro Leu Pro Asn Tyr
            580                 585                 590

GTG ACC AAA TAT GAA GTT ACT TAT AGT AGT GAG TTA GGA CCA AAC GTG        1824
Val Thr Lys Tyr Glu Val Thr Tyr Ser Ser Glu Leu Gly Pro Asn Val
        595                 600                 605

AGT GAC ACA CTT GAA AGT GAT AAA ATT TAC AAG GAT GGG ACA ATT AAA        1872
Ser Asp Thr Leu Glu Ser Asp Lys Ile Tyr Lys Asp Gly Thr Ile Lys
    610                 615                 620

TTT GAT TTT ACC AAA TAT AGT AAA AAT GAA CAA GGA TTA TTT TAT GAC        1920
Phe Asp Phe Thr Lys Tyr Ser Lys Asn Glu Gln Gly Leu Phe Tyr Asp
625                 630                 635                 640

AGT GGA TTA AAT TGG GAC TTT AAA ATT AAT GCT ATT ACT TAT GAT GGT        1968
Ser Gly Leu Asn Trp Asp Phe Lys Ile Asn Ala Ile Thr Tyr Asp Gly
                645                 650                 655

AAA GAG ATG AAT GTT TTT CAT AGA TAT AAT AAA TAG                        2004
Lys Glu Met Asn Val Phe His Arg Tyr Asn Lys
            660                 665

(2) INFORMATION FOR SEQ ID NO: 7:

        (i) SEQUENCE CHARACTERISTICS:
            (A) LENGTH: 667 amino acids
            (B) TYPE: amino acid
            (D) TOPOLOGY: linear

    (ii) MOLECULE TYPE: protein

    (xi) SEQUENCE DESCRIPTION: SEQ ID NO: 7:

Met Lys Arg Glu Ile Asp Glu Asp Thr Asp Thr Asp Gly Asp Ser Ile
1               5                   10                  15

Pro Asp Leu Trp Glu Glu Asn Gly Tyr Thr Ile Xaa Asn Arg Ile Ala
            20                  25                  30

Val Lys Trp Asp Asp Ser Leu Ala Ser Lys Gly Tyr Thr Lys Phe Val
        35                  40                  45

Ser Asn Pro Leu Glu Ser His Thr Val Gly Asp Pro Tyr Thr Asp Tyr
    50                  55                  60

Glu Lys Ala Ala Arg Asp Leu Asp Leu Ser Asn Ala Lys Glu Thr Phe
65                  70                  75                  80

Asn Pro Leu Val Ala Ala Phe Pro Ser Val Asn Val Ser Met Glu Lys
                85                  90                  95

Val Ile Leu Ser Pro Asn Glu Asn Leu Ser Asn Ser Val Glu Ser His
            100                 105                 110

Ser Ser Thr Asn Trp Ser Tyr Thr Asn Thr Glu Gly Ala Ser Val Glu
        115                 120                 125

Ala Gly Ile Gly Pro Lys Gly Ile Ser Phe Gly Val Ser Val Asn Tyr
    130                 135                 140

Gln His Ser Glu Thr Val Ala Gln Glu Trp Gly Thr Ser Thr Gly Asn
145                 150                 155                 160

Thr Ser Gln Phe Asn Thr Ala Ser Ala Gly Tyr Leu Asn Ala Asn Val
                165                 170                 175

Arg Tyr Asn Asn Val Gly Thr Gly Ala Ile Tyr Asp Val Lys Pro Thr
            180                 185                 190

Thr Ser Phe Val Leu Asn Asn Asp Thr Ile Ala Thr Ile Thr Ala Lys
        195                 200                 205

Ser Asn Ser Thr Ala Leu Asn Ile Ser Pro Gly Glu Ser Tyr Pro Lys
    210                 215                 220

Lys Gly Gln Asn Gly Ile Ala Ile Thr Ser Met Asp Asp Phe Asn Ser
225                 230                 235                 240

His Pro Ile Thr Leu Asn Lys Lys Gln Val Asp Asn Leu Leu Asn Asn
                245                 250                 255

Lys Pro Met Met Leu Glu Thr Asn Gln Thr Asp Gly Val Tyr Lys Ile
            260                 265                 270

Lys Asp Thr His Gly Asn Ile Val Thr Gly Gly Glu Trp Asn Gly Val
        275                 280                 285

Ile Gln Gln Ile Lys Ala Lys Thr Ala Ser Ile Ile Val Asp Asp Gly
    290                 295                 300

Glu Arg Val Ala Glu Lys Arg Val Ala Ala Lys Asp Tyr Glu Asn Pro
305                 310                 315                 320

Glu Asp Lys Thr Pro Ser Leu Thr Leu Lys Asp Ala Leu Lys Leu Ser
                325                 330                 335

Tyr Pro Asp Glu Ile Lys Glu Ile Glu Gly Leu Leu Tyr Tyr Lys Asn
            340                 345                 350

Lys Pro Ile Tyr Glu Ser Ser Val Met Thr Tyr Leu Asp Glu Asn Thr
        355                 360                 365

Ala Lys Glu Val Thr Lys Gln Leu Asn Asp Thr Thr Gly Lys Phe Lys
    370                 375                 380

Asp Val Ser His Leu Tyr Asp Val Lys Leu Thr Pro Lys Met Asn Val
385                 390                 395                 400

Thr Ile Lys Leu Ser Ile Leu Tyr Asp Asn Ala Glu Ser Asn Asp Asn
                405                 410                 415

Ser Ile Gly Lys Trp Thr Asn Thr Asn Ile Val Ser Gly Gly Asn Asn
            420                 425                 430

Gly Lys Lys Gln Tyr Ser Ser Asn Asn Pro Asp Ala Asn Leu Thr Leu
        435                 440                 445

Asn Thr Asp Ala Gln Glu Lys Leu Asn Lys Asn Arg Asp Tyr Tyr Ile
    450                 455                 460

Ser Leu Tyr Met Lys Ser Glu Lys Asn Thr Gln Cys Glu Ile Thr Ile
465                 470                 475                 480

Asp Gly Glu Ile Tyr Pro Ile Thr Thr Lys Thr Val Asn Val Asn Lys
                485                 490                 495

Asp Asn Tyr Lys Arg Leu Asp Ile Ile Ala His Asn Ile Lys Ser Asn
            500                 505                 510

Pro Ile Ser Ser Leu His Ile Lys Thr Asn Asp Glu Ile Thr Leu Phe
        515                 520                 525

Trp Asp Asp Ile Ser Ile Thr Asp Val Ala Ser Ile Lys Pro Glu Asn
    530                 535                 540

Leu Thr Asp Ser Glu Ile Lys Gln Ile Tyr Ser Arg Tyr Gly Ile Lys
545                 550                 555                 560

Leu Glu Asp Gly Ile Leu Ile Asp Lys Lys Gly Gly Ile His Tyr Gly
                565                 570                 575

Glu Phe Ile Asn Glu Ala Ser Phe Asn Ile Glu Pro Leu Pro Asn Tyr
            580                 585                 590

Val Thr Lys Tyr Glu Val Thr Tyr Ser Ser Glu Leu Gly Pro Asn Val
        595                 600                 605

Ser Asp Thr Leu Glu Ser Asp Lys Ile Tyr Lys Asp Gly Thr Ile Lys
    610                 615                 620

Phe Asp Phe Thr Lys Tyr Ser Lys Asn Glu Gln Gly Leu Phe Tyr Asp
625                 630                 635                 640

Ser Gly Leu Asn Trp Asp Phe Lys Ile Asn Ala Ile Thr Tyr Asp Gly
                645                 650                 655

Lys Glu Met Asn Val Phe His Arg Tyr Asn Lys
            660                 665

(2) INFORMATION FOR SEQ ID NO: 8:

    (i) SEQUENCE CHARACTERISTICS:

(A) LENGTH: 16 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus cereus
(B) STRAIN: AB78
(C) INDIVIDUAL ISOLATE: NRRL B-21058

(ix) FEATURE:
(A) NAME/KEY: Peptide
(B) LOCATION: 1..16
(D) OTHER INFORMATION: /note= "N-terminal sequence of
protein purified from strain AB78"

(xi) SEQUENCE DESCRIPTION: SEQ ID NO: 8:

Lys Arg Glu Ile Asp Glu Asp Thr Asp Thr Asx Gly Asp Ser Ile Pro
1               5                   10                  15

(2) INFORMATION FOR SEQ ID NO: 9:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(ix) FEATURE:
(A) NAME/KEY: misc_feature
(B) LOCATION: 1..21
(D) OTHER INFORMATION: /note= "Oligonucleotide probe based
on amino acids 3 to 9 of SEQ ID NO:8, using codon
usage of Bacillus thuringiensis"

(xi) SEQUENCE DESCRIPTION: SEQ ID NO: 9:

GAAATTGATC AAGATACNGA T                                                21

(2) INFORMATION FOR SEQ ID NO: 10:

(i) SEQUENCE CHARACTERISTICS:
(A) LENGTH: 14 amino acids

(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis
(B) STRAIN: AB88

(ix) FEATURE:
(A) NAME/KEY: Peptide
(B) LOCATION: 1..14
(D) OTHER INFORMATION: /note= "N-terminal amino acid
sequence of protein known as anion exchange
fraction 23 (smaller)"

(xi) SEQUENCE DESCRIPTION: SEQ ID NO: 10:

Xaa Glu Pro Phe Val Ser Ala Xaa Xaa Xaa Gln Xaa Xaa Xaa
1               5                   10

(2) INFORMATION FOR SEQ ID NO: 11:

(i) SEQUENCE CHARACTERISTICS:
(A) LENGTH: 13 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis
(B) STRAIN: AB88

(ix) FEATURE:
(A) NAME/KEY: Peptide
(B) LOCATION: 1..13
(D) OTHER INFORMATION: /note= "N-terminal amino acid
sequence of protein known as anion exchange
fraction 23 (larger)"

(xi) SEQUENCE DESCRIPTION: SEQ ID NO: 11:

Xaa Glu Tyr Glu Asn Val Glu Pro Phe Val Ser Ala Xaa
1               5                   10

(2) INFORMATION FOR SEQ ID NO: 12:

(i) SEQUENCE CHARACTERISTICS:
(A) LENGTH: 14 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thurigiensis
(B) STRAIN: AB88

(ix) FEATURE:
(A) NAME/KEY: Peptide
(B) LOCATION: 1..14
(D) OTHER INFORMATION: /note= "N-terminal sequence of 80
kDa VIP active against Agrotis ipsilon"

(xi) SEQUENCE DESCRIPTION: SEQ ID NO: 12:

Met Asn Lys Asn Asn Thr Lys Leu Pro Thr Arg Ala Leu Pro
1               5                   10

(2) INFORMATION FOR SEQ ID NO: 13:

(i) SEQUENCE CHARACTERISTICS:
(A) LENGTH: 15 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis
(B) STRAIN: AB88

(ix) FEATURE:
(A) NAME/KEY: Peptide
(B) LOCATION: 1..15
(D) OTHER INFORMATION: /note= "N-terminal amino acid
sequence of 35 kDa VIP active against Agrotis
ipsilon"

(xi) SEQUENCE DESCRIPTION: SEQ ID NO: 13:

Ala Leu Ser Glu Asn Thr Gly Lys Asp Gly Gly Tyr Ile Val Pro
1               5                   10                  15

(2) INFORMATION FOR SEQ ID NO: 14:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis

(ix) FEATURE:
(A) NAME/KEY: Peptide
(B) LOCATION: 1..9
(D) OTHER INFORMATION: /note= "N-terminal sequence of a
130 kDa delta-endotoxin"

(xi) SEQUENCE DESCRIPTION: SEQ ID NO: 14:

Met Asp Asn Asn Pro Asn Ile Asn Glu
1               5

(2) INFORMATION FOR SEQ ID NO: 15:

(i) SEQUENCE CHARACTERISTICS:
(A) LENGTH: 9 amino acids
(B) TYPE: amino acid
(C) STRANDEDNESS: single
(D) TOPOLOGY: linear

(ii) MOLECULE TYPE: peptide

(iii) HYPOTHETICAL: NO

(v) FRAGMENT TYPE: N-terminal

(ix) FEATURE:
(A) NAME/KEY: Peptide
(B) LOCATION: 1..9
(D) OTHER INFORMATION: /note= "N-terminal sequence of 80
kDa delta-endotoxin"

(xi) SEQUENCE DESCRIPTION: SEQ ID NO: 15:

Met Asp Asn Asn Pro Asn Ile Asn Glu
1               5

(2) INFORMATION FOR SEQ ID NO: 16:

(i) SEQUENCE CHARACTERISTICS:
(A) LENGTH: 11 amino acids
(B) TYPE: amino acid

(C) STRANDEDNESS: single
(D) TOPOLOGY: linear

(ii) MOLECULE TYPE: peptide

(iii) HYPOTHETICAL: NO

(v) FRAGMENT TYPE: N-terminal

(vi) ORIGINAL SOURCE:
(A) ORGANISM: Bacillus thuringiensis

(ix) FEATURE:
(A) NAME/KEY: Peptide
(B) LOCATION: 1..11
(D) OTHER INFORMATION: /note= "N-terminal sequence from 60
kDa delta-endotoxin"

(xi) SEQUENCE DESCRIPTION: SEQ ID NO: 16:

Met Asn Val Leu Asn Ser Gly Arg Thr Thr Ile
1               5                   10

(2) INFORMATION FOR SEQ ID NO: 17:

(i) SEQUENCE CHARACTERISTICS:
(A) LENGTH: 2655 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iii) ANTI-SENSE: NO

(xi) SEQUENCE DESCRIPTION: SEQ ID NO: 17:

ATGAAGAACA TGAAGAAGAA GCTGGCCAGC GTGGTGACCT GCACCCTGCT GGCCCCCATG 60

TTCCTGAACG GCAACGTGAA CGCCGTGTAC GCCGACAGCA AGACCAACCA GATCAGCACC 120

ACCCAGAAGA ACCAGCAGAA GGAGATGGAC CGCAAGGGCC TGCTGGGCTA CTACTTCAAG 180

GGCAAGGACT TCAGCAACCT GACCATGTTC GCCCCCACGC GTGACAGCAC CCTGATCTAC 240

GACCAGCAGA CCGCCAACAA GCTGCTGGAC AAGAAGCAGC AGGAGTACCA GAGCATCCGC 300

TGGATCGGCC TGATCCAGAG CAAGGAGACC GGCGACTTCA CCTTCAACCT GAGCGAGGAC 360

GAGCAGGCCA TCATCGAGAT CAACGGCAAG ATCATCAGCA ACAAGGGCAA GGAGAAGCAG 420

GTGGTGCACC TGGAGAAGGG CAAGCTGGTG CCCATCAAGA TCGAGTACCA GAGCGACACC 480

AAGTTCAACA TCGACAGCAA GACCTTCAAG GAGCTGAAGC TTTTCAAGAT CGACAGCCAG 540

AACCAGCCCC AGCAGGTGCA GCAGGACGAG CTGCGCAACC CCGAGTTCAA CAAGAAGGAG 600
AGCCAGGAGT TCCTGGCCAA GCCCAGCAAG ATCAACCTGT TCACCCAGCA GATGAAGCGC 660
GAGATCGACG AGGACACCGA CACCGACGGC GACAGCATCC CCGACCTGTG GGAGGAGAAC 720
GGCTACACCA TCCAGAACCG CATCGCCGTG AAGTGGGACG ACAGCCTGGC TAGCAAGGGC 780
TACACCAAGT TCGTGAGCAA CCCCCTGGAG AGCCACACCG TGGGCGACCC CTACACCGAC 840
TACGAGAAGG CCGCCCGCGA CCTGGACCTG AGCAACGCCA AGGAGACCTT CAACCCCCTG 900
GTGGCCGCCT TCCCCAGCGT GAACGTGAGC ATGGAGAAGG TGATCCTGAG CCCCAACGAG 960
AACCTGAGCA ACAGCGTGGA GAGCCACTCG AGCACCAACT GGAGCTACAC CAACACCGAG 1020
GGCGCCAGCG TGGAGGCCGG CATCGGTCCC AAGGGCATCA GCTTCGGCGT GAGCGTGAAC 1080
TACCAGCACA GCGAGACCGT GGCCCAGGAG TGGGGCACCA GCACCGGCAA CACCAGCCAG 1140
TTCAACACCG CCAGCGCCGG CTACCTGAAC GCCAACGTGC GCTACAACAA CGTGGGCACC 1200
GGCGCCATCT ACGACGTGAA GCCCACCACC AGCTTCGTGC TGAACAACGA CACCATCGCC 1260
ACCATCACCG CCAAGTCGAA TTCCACCGCC CTGAACATCA GCCCCGGCGA GAGCTACCCC 1320
AAGAAGGGCC AGAACGGCAT CGCCATCACC AGCATGGACG ACTTCAACAG CCACCCCATC 1380
ACCCTGAACA AGAAGCAGGT GGACAACCTG CTGAACAACA AGCCCATGAT GCTGGAGACC 1440
AACCAGACCG ACGGCGTCTA CAAGATCAAG GACACCCACG GCAACATCGT GACCGGCGGC 1500
GAGTGGAACG GCGTGATCCA GCAGATCAAG GCCAAGACCG CCAGCATCAT CGTCGACGAC 1560
GGCGAGCGCG TGGCCGAGAA GCGCGTGGCC GCCAAGGACT ACGAGAACCC CGAGGACAAG 1620
ACCCCCAGCC TGACCCTGAA GGACGCCCTG AAGCTGAGCT ACCCCGACGA GATCAAGGAG 1680
ATCGAGGGCC TGCTGTACTA CAAGAACAAG CCCATCTACG AGAGCAGCGT GATGACCTAT 1740
CTAGACGAGA ACACCGCCAA GGAGGTGACC AAGCAGCTGA ACGACACCAC CGGCAAGTTC 1800
AAGGACGTGA GCCACCTGTA CGACGTGAAG CTGACCCCCA AGATGAACGT GACCATCAAG 1860
CTGAGCATCC TGTACGACAA CGCCGAGAGC AACGACAACA GCATCGGCAA GTGGACCAAC 1920
ACCAACATCG TGAGCGGCGG CAACAACGGC AAGAAGCAGT ACAGCAGCAA CAACCCCGAC 1980
GCCAACCTGA CCCTGAACAC CGACGCCCAG GAGAAGCTGA ACAAGAACCG CGACTACTAC 2040
ATCAGCCTGT ACATGAAGAG CGAGAAGAAC ACCCAGTGCG AGATCACCAT CGACGGCGAG 2100
ATATACCCCA TCACCACCAA GACCGTGAAC GTGAACAAGG ACAACTACAA GCGCCTGGAC 2160
ATCATCGCCC ACAACATCAA GAGCAACCCC ATCAGCAGCC TGCACATCAA GACCAACGAC 2220
GAGATCACCC TGTTCTGGGA CGACATATCG ATTACCGACG TCGCCAGCAT CAAGCCCGAG 2280
AACCTGACCG ACAGCGAGAT CAAGCAGATA TACAGTCGCT ACGGCATCAA GCTGGAGGAC 2340

GGCATCCTGA TCGACAAGAA GGGCGGCATC CACTACGGCG AGTTCATCAA CGAGGCCAGC 2400
TTCAACATCG AGCCCCTGCA GAACTACGTG ACCAAGTACG AGGTGACCTA CAGCAGCGAG 2460
CTGGGCCCCA ACGTGAGCGA CACCCTGGAG AGCGACAAGA TTTACAAGGA CGGCACCATC 2520
AAGTTCGACT TCACCAAGTA CAGCAAGAAC GAGCAGGGCC TGTTCTACGA CAGCGGCCTG 2580
AACTGGGACT TCAAGATCAA CGCCATCACC TACGACGGCA AGGAGATGAA CGTGTTCCAC 2640
CGCTACAACA AGTAG 2655
(2) INFORMATION FOR SEQ ID NO: 18:
(i) SEQUENCE CHARACTERISTICS:
(A) LENGTH: 2010 base pairs
(B) TYPE: nucleic acid
(C) STRANDEDNESS: single
(D) TOPOLOGY: linear
(ii) MOLECULE TYPE: DNA (genomic)
(iii) HYPOTHETICAL: NO
(iii) ANTI-SENSE: NO
(xi) SEQUENCE DESCRIPTION: SEQ ID NO: 18:
GGATCCATGA AGCGCGAGAT CGACGAGGAC ACCGACACCG ACGGCGACAG CATCCCCGAC 60
CTGTGGGAGG AGAACGGCTA CACCATCCAG AACCGCATCG CCGTGAAGTG GGACGACAGC 120
CTGGCTAGCA AGGGCTACAC CAAGTTCGTG AGCAACCCCC TGGAGAGCCA CACCGTGGGC 180
GACCCCTACA CCGACTACGA GAAGGCCGCC CGCGACCTGG ACCTGAGCAA CGCCAAGGAG 240
ACCTTCAACC CCCTGGTGGC CGCCTTCCCC AGCGTGAACG TGAGCATGGA GAAGGTGATC 300
CTGAGCCCCA ACGAGAACCT GAGCAACAGC GTGGAGAGCC ACTCGAGCAC CAACTGGAGC 360
TACACCAACA CCGAGGGCGC CAGCGTGGAG GCCGGCATCG GTCCCAAGGG CATCAGCTTC 420
GGCGTGAGCG TGAACTACCA GCACAGCGAG ACCGTGGCCC AGGAGTGGGG CACCAGCACC 480
GGCAACACCA GCCAGTTCAA CACCGCCAGC GCCGGCTACC TGAACGCCAA CGTGCGCTAC 540
AACAACGTGG GCACCGGCGC CATCTACGAC GTGAAGCCCA CCACCAGCTT CGTGCTGAAC 600
AACGACACCA TCGCCACCAT CACCGCCAAG TCGAATTCCA CCGCCCTGAA CATCAGCCCC 660
GGCGAGAGCT ACCCCAAGAA GGGCCAGAAC GGCATCGCCA TCACCAGCAT GGACGACTTC 720
AACAGCCACC CCATCACCCT GAACAAGAAG CAGGTGGACA ACCTGCTGAA CAACAAGCCC 780
ATGATGCTGG AGACCAACCA GACCGACGGC GTCTACAAGA TCAAGGACAC CCACGGCAAC 840
ATCGTGACCG GCGGCGAGTG GAACGGCGTG ATCCAGCAGA TCAAGGCCAA GACCGCCAGC 900

ATCATCGTCG ACGACGGCGA GCGCGTGGCC GAGAAGCGCG TGGCCGCCAA GGACTACGAG 960

AACCCCGAGG ACAAGACCCC CAGCCTGACC CTGAAGGACG CCCTGAAGCT GAGCTACCCC 1020

GACGAGATCA AGGAGATCGA GGGCCTGCTG TACTACAAGA ACAAGCCCAT CTACGAGAGC 1080

AGCGTGATGA CCTATCTAGA CGAGAACACC GCCAAGGAGG TGACCAAGCA GCTGAACGAC 1140

ACCACCGGCA AGTTCAAGGA CGTGAGCCAC CTGTACGACG TGAAGCTGAC CCCCAAGATG 1200

AACGTGACCA TCAAGCTGAG CATCCTGTAC GACAACGCCG AGAGCAACGA CAACAGCATC 1260

GGCAAGTGGA CCAACACCAA CATCGTGAGC GGCGGCAACA ACGGCAAGAA GCAGTACAGC 1320

AGCAACAACC CCGACGCCAA CCTGACCCTG AACACCGACG CCCAGGAGAA GCTGAACAAG 1380

AACCGCGACT ACTACATCAG CCTGTACATG AAGAGCGAGA AGAACACCCA GTGCGAGATC 1440

ACCATCGACG GCGAGATATA CCCCATCACC ACCAAGACCG TGAACGTGAA CAAGGACAAC 1500

TACAAGCGCC TGGACATCAT CGCCCACAAC ATCAAGAGCA ACCCCATCAG CAGCCTGCAC 1560

ATCAAGACCA ACGACGAGAT CACCCTGTTC TGGGACGACA TATCGATTAC CGACGTCGCC 1620

AGCATCAAGC CCGAGAACCT GACCGACAGC GAGATCAAGC AGATATACAG TCGCTACGGC 1680

ATCAAGCTGG AGGACGGCAT CCTGATCGAC AAGAAGGGCG GCATCCACTA CGGCGAGTTC 1740

ATCAACGAGG CCAGCTTCAA CATCGAGCCC CTGCAGAACT ACGTGACCAA GTACGAGGTG 1800

ACCTACAGCA GCGAGCTGGG CCCCAACGTG AGCGACACCC TGGAGAGCGA CAAGATTTAC 1860

AAGGACGGCA CCATCAAGTT CGACTTCACC AAGTACAGCA AGAACGAGCA GGGCCTGTTC 1920

TACGACAGCG GCCTGAACTG GGACTTCAAG ATCAACGCCA TCACCTACGA CGGCAAGGAG 1980

ATGAACGTGT TCCACCGCTA CAACAAGTAG 2010

## Description

The present invention is a continuation-in-part application of U.S. application serial number 08/037,057 filed March 25, 1993, the disclosures of which are herein incorporated by reference.

### FIELD OF THE INVENTION

The present invention is drawn to methods and compositions for controlling plant and non-plant pests.

### BACKGROUND OF THE INVENTION

Insect pests are a major factor in the loss of the world's commercially important agricultural crops. Broad spectrum chemical pesticides have been used extensively to control or eradicate pests of agricultural importance. There is, however, substantial interest in developing effective alternative pesticides.

Microbial pesticides have played an important role as alternatives to chemical pest control. The most extensively used microbial product is based on the bacterium Bacillus thuringiensis (Bt). Bt is a gram-positive spore forming Bacillus which produces an insecticidal crystal protein (ICP) during sporulation.

Numerous varieties of Bt are known that produce more than 25 different but related ICP's. The ICP's made by Bt are toxic to larvae of certain insects in the orders Lepidoptera, Diptera and Coleoptera. In general, when the ICP is ingested by a susceptible insect the crystal is solubilized and transformed into a toxic moiety by the insect gut proteases. None of the ICP's active against coleopteran larvae have demonstrated significant effects on the genus Diabrotica particularly Diabrotica virgifera virgifera, the western com rootworm (WCRW) or Diabrotica longicornis barberi, the northern com rootworm.

Bt is closely related to Bacillus cereus (Bc). A major distinguishing characteristic is the lack of a parasporal crystal in Bc. Bc is a widely distributed bacterium that is commonly found in soil and has been isolated from a variety of foods and drugs. The organism has been implicated in the spoilage of food.

Although Bt has been very useful in controlling insect pests, there is a need to expand the number of potential biological control agents.

### SUMMARY OF THE INVENTION

The present invention is drawn to compositions and methods for controlling plant and non-plant pests. Particularly, new pesticidal proteins are disclosed which are isolatable from the vegetative growth stage of Bacillus. Bacillus strains, proteins, and genes encoding the proteins are provided.

The methods and compositions of the invention may be used in a variety of systems for controlling plant and non-plant pests.

### DETAILED DESCRIPTION OF THE INVENTION

Compositions and methods for controlling plant pests are provided. In particular, novel pesticidal proteins are provided which are produced during vegetative growth of Bacillus strains. The proteins are useful as pesticidal agents.

The present invention recognizes that pesticidal proteins are produced during vegetative growth of Bacillus strains. For the purpose of the present invention vegetative growth is defined as that period of time before the onset of sporulation. In the case of Bt, this vegetative growth occurs before production of ICPs. Genes encoding such proteins can be isolated, cloned and transformed into various delivery vehicles for use in pest management programs.

For purposes of the present invention, pests include but are not limited to insects, fungi, bacteria, nematodes, mites, ticks, protozoan pathogens, animal-parasitic liver flukes, and the like. Insect pests include insects selected from the orders Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera, etc.

Tables 1 - 10 gives a list of pests associated with major crop plants and pests of human and veterinary importance. Such pests are included within the scope of the present invention.

**TABLE 1**

| Lepidoptera (Butterflies and Moths) | |
|---|---|
| Maize | Sunflower |
| Ostrinia nubilalis, European corn borer | Suleima helianthana, sunflower bud moth |
| Agrotis ipsilon, black cutworm | Homoeosoma electellum, sunflower moth |
| Helicoverpa zea, corn earworm | Cotton |
| Spodoptera frugiperda, fall armyworm | Heliothis virescens, cotton boll worm |
| Diatraea grandiosella, southwestern corn | Helicoverpa zea, cotton bollworm |
| borer | Spodoptera exigua, beet armyworm |
| Elasmopalpus lignosellus, lesser cornstalk | Pectinophora gossypiella, pink bollworm |
| borer | Rice |
| Diatraea saccharalis, sugarcane borer | Diatraea saccharalis, sugarcane borer |
| Sorghum | Spodoptera frugiperda, fall armyworm |
| Chilo partellus, sorghum borer | Helicoverpa zea, corn earworm |
| Spodoptera frugiperda, fall armyworm | Soybean |
| Helicoverpa zea, corn earworm | Pseudoplusia includens, soybean looper |
| Elasmopalpus lignosellus, lesser cornstalk | Anticarsia gemmatalis, velvetbean |
| borer | caterpillar |
| Feltia subterranea, granulate cutworm | Plathypena scabra, green cloverworm |
| Wheat | Ostrinia nubilalis, European corn borer |
| Pseudaletia unipunctata, army worm | Agrotis ipsilon, black cutworm |
| Spodoptera frugiperda, fall armyworm | Spodoptera exigua, beet armyworm |
| Elasmopalpus lienosellus, lesser cornstalk | Heliothis virescens, cotton boll worm |
| borer | Helicoverpa zea, cotton bollworm |
| Agrotis orthogonia, pale western cutworm | Barley |
| Elasrnopalpus lignosellus, lesser cornstalk | Ostrinia nubilalis, European corn borer |
| borer | Agrotis ipsilon, black cutworm |

**TABLE 2**

| Coleoptera (Beetles) |
|---|
| Maize |
| Diabrotica virgifera virgifera, western corn rootworm |
| Diabrotica longicornis barberi, northern corn rootworm |
| Diabrotica undecimpunctata howardi, southern corn rootworm |
| Melanotus spp., wireworms |
| Cyclocenhala borealis, northern masked chafer (white grub) |
| Cyclocenhala immaculata, southern masked chafer (white grub) |
| Popillia japonica, Japanese beetle |
| Chaetocnema pulicaria, corn flea beetle |
| Sphenophorus maidis, maize billbug |
| |

| Sorghum |
|---|
| Phyllophaga crinita, white grub |
| Eleodes, Conoderus, and Aeolus spp., wireworms |
| Oulema melanopus, cereal leaf beetle |
| Chaetocnema pulicaria, corn flea beetle |
| Sphenophorus maidis, maize billbug |
| |

| Wheat |
|---|
| Oulema melanopus, cereal leaf beetle |
| Hypera punctata, clover leaf weevil |
| Diabrotica undecimpunctata howardi, southern corn rootworm |
| |

| Sunflower |
|---|
| Zygogramma exclamationis, sunflower beetle |
| Bothyrus gibbosus, carrot beetle |
| |

| Cotton |
|---|
| Anthonomus grandis, boll weevil |
| |

| Rice |
|---|
| Colaspis brunnea, grape colaspis |
| Lissorhoptrus oryzophilus, rice water weevil |
| Sitophilus oryzae, rice weevil |
| |

| Soybean |
|---|
| Epilachna varivestis, Mexican bean beetle |

**TABLE 3**

| Homoptera (Whiteflies. Aphids etc..) |
|---|
| Maize |
| Rhopalosiphum maidis, corn leaf aphid |
| Anuraphis maidiradicis, corn root aphid |
| |

| Sorghum |
|---|
| Rhopalosiphum maidis, corn leaf aphid |
| Sipha flava, yellow sugarcane aphid |
| |

| Wheat |
|---|
| Russian wheat aphid |
| Schizaphis graminum, greenbug |
| Macrosiphum avenae, English grain aphid |
| |

| Cotton |
|---|
| Aphis gossypii, cotton aphid |
| Pseudatomoscelis seriatus, cotton fleahopper |
| Trialeurodes abutilonea, bandedwinged whitefly |
| |

| Rice |
|---|
| Nephotettix nigropictus, rice leafhopper |
| |

| Soybean |
|---|
| Myzus persicae, green peach aphid |
| Empoasca fabae, potato leafhopper |
| |

| Barley |
|---|
| Schizaphis graminum, greenbug |
| |

| Oil Seed Rape |
|---|
| Brevicoryne brassicae, cabbage aphid |

**TABLE 4**

| Hemiptera (Bugs) |
|---|
| Maize |
| Blissus leucopterus leucopterus, chinch bug |
| |

| Sorghum |
|---|
| Blissus leucopterus leucopterus, chinch bug |
| |

| Cotton |
|---|
| Lygus lineolaris, tarnished plant bug |
| |

| Rice |
|---|
| Blissus leucopterus leucopterus, chinch bug |
| Acrosternum hilare, green stink bug |
| |

| Soybean |
|---|
| Acrosternum hilare, green stink bug |
| |

| Barley |
|---|
| Blissus leucopterus Leucopterus, chinch bug |
| Acrosternum hilare, green stink bug |
| Euschistus servus, brown stink bug |

**TABLE 5**

| Orthoptera (Grasshoppers. Crickets. and Cockroaches) |
|---|
| Maize |
| Melanoplus femurrubrum, redlegged grasshopper |
| Melanoplus sanguinipes, migratory grasshopper |
| |

| Wheat |
|---|
| Melanoplus femurrubrum, redlegged grasshopper |
| Melanoplus differentialis, differential grasshopper |
| Melanoplus sanguinipes, migratory grasshopper |
| |

| Cotton |
|---|
| Melanoplus femurrubrum, redlegged grasshopper |
| Melanoplus differentialis, differential grasshopper |
| |

| Soybean |
|---|
| Melanoplus femurrubrum, redlegged grasshopper |
| Melanoplus differentialis, differential grasshopper |
| |

| Structural/Household |
|---|
| Periplaneta americana, American cockroach |
| Blattella germanica, German cockroach |
| Blatta orientalis, oriental cockroach |

**TABLE 6**

| Diptera (Flies and Mosquitoes) |
|---|
| Maize |
| Hylemya platura, seedcorn maggot |
| Agromyza parvicomis, corn blotch leafrniner |
| |

| Sorghum |
|---|
| Contarinia sorphicola, sorghum midge |
| |

| Wheat |
|---|
| Mayetiola destructor, Hessian fly |
| Sitodiplosis mosellana, wheat midge |
| Meromyza americana, wheat stem maggot |
| Hylemya coarctata, wheat bulb fly |
| |

| Sunflower |
|---|
| Neolasioptera murtfeldtiana, sunflower seed midge |
| |

| Soybean |
|---|
| Hylemya platura, seedcorn maggot |
| |

| Barley |
|---|
| Hylemya platura, seedcorn maggot |
| Mayetiola destructor, Hessian fly |
| |

| Insects attacking humans and animals and disease carriers |
|---|
| Aedes aegypti, yellowfever mosquito |
| Aedes albopictus, forest day mosquito |
| Phlebotomus papatasii, sand fly |
| Musca domestica, house fly |
| Tabanus atratus, black horse fly |
| Cochliomyia hominivorax, screwworm fly |

**TABLE 7**

| Thysanoptera (Thrips) |
|---|
| Maize |
| Anaphothrips obscurus, grass thrips |
| |

| Wheat |
|---|
| Frankliniella fusca, tobacco thrips |
| |

| Cotton |
|---|
| Thrips tabaci, onion thrips |
| Frankliniella fusca, tobacco thrips |
| |

| Soybean |
|---|
| Sericothrips variabilis, soybean thrips |
| Thrips tabaci, onion thrips |

**TABLE 8**

| Hymenoptera (Sawflies, Ants, Wasps, etc.) |
|---|
| Maize |
| Solenopsis milesta, thief ant |
| |

| Wheat |
|---|
| Cephus cinctus, wheat stem sawfly |

**TABLE 9**

| Other Orders and Representative Species |
|---|
| Dermaptera (Earwigs) |
| Forficula auricularia, European earwig |
| |

| Isoptera (Termites) |
|---|
| Reticulitermes flavipes, eastern subterranean termite |
| |

| Mallophaga (Chewing Lice) |
|---|
| Cuclotogast heterogranha, chicken head louse |
| Bovicola bovis, cattle biting louse |
| |

| Anoplura (Sucking Lice) |
|---|
| Pediculus humanus, head and body louse |
| |

| Siphonaptera (Fleas) |
|---|
| Ctenocephalides felis, cat flea |

**TABLE 10**

| Acari (Mites and Ticks) |
|---|
| Maize |
| Tetranychus urticae, twospotted spider mite |
| |

| Sorghum |
|---|
| Tetranychus cinnabarinus, carmine spider mite |
| Tetranychus urticae, twospotted spider mite |
| |

| Wheat |
|---|
| Aceria tulipae, wheat curl mite |
| |

| Cotton |
|---|
| Tetranychus cinnabarinus, carmine spider mite |
| Tetranychus urticae, twospotted spider mite |
| |

| Soybean |
|---|
| Tetranychus turkestani, strawberry spider mite |
| Tetranychus urticae, twospotted spider mite |
| |

| Barley |
|---|
| Petrobia latens, brown wheat mite |
| |

| Important human and animal Acari |
|---|
| Demacentor variabilis, American dog tick |
| Argas persicus, fowl tick |
| Dermatophagoides farinae, American house dust mite |
| Dermatophagoides pteronyssinus, European house dust mite |

Now that it has been recognized that pesticidal proteins can be isolated from the vegetative growth phase of Bacillus, other strains can be isolated by standard techniques and tested for activity against particular plant and non-plant pests. Generally Bacillus strains can be isolated from any environmental sample, including soil, plant, insect, grain elevator dust, and other sample material, etc., by methods known in the art. See, for example, Travers et al. (1987) Appl. Environ. Microbiol. 53:1263-1266; Saleh et al. (1969) Can J. Microbiol. 15:1101-1104; DeLucca et al. (1981) Can J. Microbiol. 27:865-870; and Norris, et al. (1981) "The genera Bacillus and Sporolactobacillus," In Starr et al. (eds.), The Prokaryotes: A Handbook on Habitats, Isolation, and Identification of Bacteria, Vol. II, Springer-Verlog Berlin Heidelberg. After isolation, strains can be tested for pesticidal activity during vegetative growth. In this manner, new pesticidal proteins and strains can be identified.

Such Bacillus microorganisms which find use in the invention include Bacillus cereus and Bacillus thuringiensis, as well as those Bacillus species listed in Table 11.

**TABLE 11**

| List of Bacillus species | |
|---|---|
| Morphological Group 1 | **Unassigned Strains** |
| B. megaterium | Subgroup A |
| B. cereus* | B. apiarus* |
| B. cereus var. mycoides | B. filicolonicus |
| B. thuringiensis* | B. thiaminolyticus |
| B. licheniformis | B. alcalophilus |
| B. subtilis* | |
| B. pumilus | Subgroup B |
| B. firmus* | B. cirroflagellosus |
| B. coagulans | B. chitinosporus |
| | B. lentus |
| Morphological Group 2 | |
| B. polymyxa | Subgroup C |
| B. macerans | B. badius |
| B. circulans | B. aneurinolyticus |
| B. stearothermophilus | B. macroides |
| B. alvei* | B. freundenreichii |
| B. laterosporus* B. brevis | |
| B. pulvifaciens | Subgroup D |
| B. popilliae* | B. pantothenticus |
| B. lentimorbus* | B. epiphytus |
| B. larvae* | |
| | Subgroup E1 |
| Morphological Group 3 | B. aminovorans |
| B. sphaericus* | B. globisporus |
| B. pasteurii | B. insolitus |
| | B. psychrophilus |
| | |
| | Subgroup E2 |
| | B. psychrosaccharolvticus |
| | B. macquariensis |

| | |
|---|---|
| *=Those Bacillus strains that have been Grouping according to Parry, J.M. et al. Publications, London. previously (1983) found in insects Color Atlas of Bacillus species, Wolfe Medical Publications, London. | |

In accordance with the present invention, the pesticidal proteins produced during vegetative growth can be isolated from Bacillus. In one embodiment, insecticidal proteins produced during vegetative growth, herein after referred to as VIP's (Vegetative Insecticidal Protein), can be isolated. Methods for protein isolation are known in the art. Generally, proteins can be purified by conventional chromatography, including gel-filtration, ion-exchange, and immunoaffinity chromatography, by high-performance liquid chromatography, such as reversed-phase high-performance liquid chromatography, ion-exchange high-performance liquid chromatography, size-exclusion high-performance liquid chromatography, high-performance chromatofocusing and hydrophobic interaction chromatography, etc., by electrophoretic separation, such as one-dimensional gel electrophoresis, two-dimensional gel electrophoresis, etc. Such methods are known in the art. See for example Current Protocols in Molecular Biology, Vols. 1 and 2, Ausubel et al. (eds.), John Wiley & Sons, NY (1988). Additionally, antibodies can be prepared against substantially pure preparations of the protein. See, for example, Radka et al. (1983) J. Immunol. 128:2804; and Radka et al. (1984) Immunogenetics 19:63. Any combination of methods may be utilized to purify protein having pesticidal properties. As the protocol is being formulated, pesticidal activity is determined after each purification step.

Such purification steps will result in a substantially purified protein fraction. By "substantially purified" or "substantially pure" is intended protein which is substantially free of any compound normally associated with the protein in its natural state. "Substantially pure" preparations of protein can be assessed by the absence of other detectable protein bands following SDS-PAGE as determined visually or by densitometry scanning. Alternatively, the absence of other amino-terminal sequences or N-terminal residues in a purified preparation can indicate the level of purity. Purity can be verified by rechromatography of "pure" preparations showing the absence of other peaks by ion exchange, reverse phase or capillary electrophoresis. The terms "substantially pure" or "substantially purified" are not meant to exclude artificial or synthetic mixtures of the proteins with other compounds. The terms are also not meant to exclude the presence of minor impurities which do not interfere with the biological activity of the protein, and which may be present, for example, due to incomplete purification.

Some proteins are single polypeptide chains while many proteins consist of more than one polypeptide chain. Once purified protein is isolated, the protein, or the polypeptides of which it is comprised, can be characterized and sequenced by standard methods known in the art. For example, the purified protein, or the polypeptides of which it is comprised, may be fragmented as with cyanogen bromide, or with proteases such as papain, chymotrypsin, trypsin, lysyl-C endopeptidase, etc. (Oike et al. (1982) J. Biol. Chem. 257:9751-9758; Liu et al. (1983) Int. J. Pept. Protein Res. 21:209-215). The resulting peptides are separated, preferably by HPLC, or by resolution of gels and electroblotting onto PVDF membranes, and subjected to amino acid sequencing. To accomplish this task, the peptides are preferably analyzed by automated sequenators. It is recognized that N-terminal, C-terminal, or internal amino acid sequences can be determined. From the amino acid sequence of the purified protein, a nucleotide sequence can be synthesized which can be used as a probe to aid in the isolation of the gene encoding the pesticidal protein.

It is recognized that the proteins will vary in molecular weight, component peptides, activity against particular pests, and in other characteristics. However, by the methods set forth herein, proteins active against a variety of pests may be isolated and characterized.

Once the purified protein has been isolated and characterized it is recognized that it may be altered in various ways including amino acid substitutions, deletions, and insertions. Methods for such manipulations are generally known in the art. For example, amino acid sequence variants of the pesticidal proteins can be prepared by mutations in the DNA. Such variants will possess the desired pesticidal activity. Obviously, the mutations that will be made in the DNA encoding the variant must not place the sequence out of reading frame and preferably will not create complementary regions that could produce secondary mRNA structure. See, EP Patent Application Publication No. 75,444.

In this manner, the present invention encompasses the pesticidal proteins as well as components and fragments thereof. That is, it is recognized that component polypeptides or fragments of the proteins may be produced which retain pesticidal activity. These fragments include truncated sequences, as well as N-terminal, C-tenninal, internal and internally deleted amino acid sequences of the proteins.

Most deletions, insertions, and substitutions of the protein sequence are not expected to produce radical changes in the characteristics of the pesticidal protein. However, when it is difficult to predict the exact effect of the substitution, deletion, or insertion in advance of doing so, one skilled in the art will appreciate that the effect will be evaluated by routine screening assays.

The proteins or other component polypeptides described herein may be used alone or in combination. That is, several proteins may be used to control different insect pests. Additionally, certain of the proteins of the invention enhance the activity of the pesticidal proteins. These proteins are referred to herein as "auxiliary proteins." While the mechanism of action is not entirely certain, when the auxiliary protein and the pesticidal protein of interest are together, the insecticidal properties of the pesticidal protein are enhanced several fold.

The pesticidal proteins of the present invention may vary in molecular weight, having component polypeptides at least a molecular weight of 30 kDa or greater, preferably about 50 kDa or greater.

The auxiliary proteins of the invention may vary in molecular weight, having at least a molecular weight of about 15 kDa or greater, preferably about 20 kDa or greater. The auxiliary proteins themselves may have component polypeptides.

It is possible that the pesticidal protein and the auxilary protein may be components of a multimeric, pesticidal protein. Such a pesticidal protein which includes the auxiliary proteins as one or more of its component polypeptides may vary in molecular weight, having at least a molecular weight of 50 kDa up to at least 200 kDa, preferably about 100 kDa to 150 kDa.

An auxiliary protein may be used in combination with the pesticidal proteins of the invention to enhance activity. To determine whether the auxiliary protein will affect activity, the pesticidal protein can be expressed alone and in combination with the auxiliary protein and the respective activities compared in feeding assays for increased pesticidal activity.

It may be beneficial to screen strains for potential pesticidal activity by testing activity of the strain alone and in combination with the auxiliary protein. In some instances the auxiliary protein with the native proteins of the strains yields pesticidal activity where none is seen in the absence of the auxiliary protein.

The auxiliary protein can be modified, as described above, by various methods known in the art. Therefore, for purposes of the invention, the term "Vegetative Insecticidal Protein" (VIP) encompasses those proteins produced during vegetative growth which alone or in combination can be used for pesticidal activity. This includes pesticidal proteins, auxiliary proteins and those proteins which demonstrate activity only in the presence of the auxiliary protein or the polypeptide components of these proteins.

It is recognized that there are alternative methods available to obtain the nucleotide and amino acid sequences of the present proteins. For example, to obtain the nucleotide sequence encoding the pesticidal protein, cosmid clones, which express the pesticidal protein, can be isolated from a genomic library. From larger active cosmid clones, smaller subclones can be made and tested for activity. In this manner, clones which express an active pesticidal protein can be sequenced to determine the nucleotide sequence of the gene. Then, an amino acid sequence can be deduced for the protein. For general molecular methods, see, for example, Molecular Cloning, A Laboratory Manual, Second Edition, Vols. 1-3, Sambrook et al. (eds.) Cold Spring Harbor Laboratory Press, Cold Spring Harbor, NY (1989), and the references cited therein.

The present invention also encompasses nucleotide sequences from organisms other than Bacillus, where the nucleotide sequences are isolatable by hybridization with the Bacillus nucleotide sequences of the invention. Such nucleotide sequences can be tested for pesticidal activity. The invention also encompasses the proteins encoded by the nucleotide sequences. Furthermore, the invention encompasses proteins obtained from organisms other than Bacillus wherein the protein cross-reacts with antibodies raised against the proteins of the invention. Again the isolated proteins can be assayed for pesticidal activity by the methods disclosed herein.

Once the nucleotide sequences encoding the pesticidal proteins of the invention have been isolated, they can be manipulated and used to express the protein in a variety of hosts including other organisms, including microorganisms and plants.

The pesticidal genes of the invention can be optimized for enhanced expression in plants. See, for example U.S. Application Serial No. 07/951,715; EPA 0359472; EPA 0385962; WO 91/16432; Perlak et al (1991) Proc. Natl. Acad. Sci. USA 88:3324-3328; and Murray et al (1989) Nucleic Acids Research 17: 477-498. In this manner, the genes can be synthesized utilizing plant preferred codons. That is the preferred codon for a particular host is the single codon which most frequently encodes that amino acid in that host. The maize preferred codon, for example, for a particular amino acid may be derived from known gene sequences from maize. Maize codon usage for 28 genes from maize plants is found in Murray et al. (1989), Nucleic Acids Research 17:477-498, the disclosure of which is incorporated herein by reference. Synthetic genes could also be made based on the distribution of codons a particular host uses for a particular amino acid.

In this manner, the nucleotide sequences can be optimized for expression in any plant. It is recognized that all or any part of the gene sequence may be optimized or synthetic. That is, synthetic or partially optimized sequences may also be used.

In like manner, the nucleotide sequences can be optimized for expression in any microorganism. For Bacillus preferred codon usage, see, for example US Patent No. 5,024,837 and Johansen et al (1988) Gene 65:293-304.

Methodologies for the construction of plant expression cassettes as well as the introduction of foreign DNA into plants are described in the art. Such expression cassettes may include promoters, terminators, enhancers, leader sequences, introns and other regulatory sequences operably linked to the pesticidal protein coding sequence.

Generally, for the introduction of foreign DNA into plants Ti plasmid vectors have been utilized for the delivery of foreign DNA as well as direct DNA uptake, liposomes, electroporation, micro-injection, and the use of microprojectiles. Such methods had been published in the art. See, for example, Guerche et al., (1987) Plant Science 52:111-116; Neuhause et al., (1987) Theor. Appl. Genet. 75:30-36; Klein et al., (1987) Nature 327: 70-73; Howell et al., (1980) Science 208:1265; Horsch et al., (1985) Science 227: 1229-1231; DeBlock et al., (1989) Plant Physiology 91:694-701; Methods for Plant Molecular Biology (Weissbach and Weissbach, eds.) Academic Press, Inc. (1988); and Methods in Plant Molecular Biology (Schuler and Zielinski, eds.) Academic Press, Inc. (1989). See also US patent Application Serial No. 08/008,374 herein incorporated by reference. See also, EPA 0193259 and EPA 0451878A1. It is understood that the method of transformation will depend upon the plant cell to be transformed.

It is further recognized that the components of the expression cassette may be modified to increase expression. For example, truncated sequences, nucleotide substitutions or other modifications may be employed. See, for example Perlak et al. (1991) Proc. Natl. Acad. Sci. USA 88:3324-3328; Murray et al. (1989) Nucleic Acids Research 17:477-498; and WO 91/16432.

The construct may also include any other necessary regulators such as terminators,(Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639); plant translational consensus sequences (Joshi, C.P., (1987), Nucleic Acids Research, 15:6643-6653), introns (Luehrsen and Walbot, (1991), Mol. Gen. Genet., 225:81-93) and the like, operably linked to the nucleotide sequence. It may be beneficial to include 5' leader sequences in the expression cassette construct. Such leader sequences can act to enhance translation. Translational leaders are known in the art and include:
Picornavirus leaders, for example, EMCV leader (Encephalomyocarditis 5' noncoding region) (Elroy-Stein, O., Fuerst, T.R., and Moss, B. (1989) PNAS USA 86:6126-6130);
Potyvirus leaders, for example, TEV leader (Tobacco Etch Virus) (Allison et al., (1986); MDMV leader (Maize Dwarf Mosaic Virus); Virology, 154:9-20), and
Human immunoglobulin heavy-chain binding protein (BiP), (Macejak, D.G., and Samow, P., (1991), Nature, 353:90-94;
Untranslated leader from the coat protein mRNA of alfalfa mosaic virus (AMV RNA 4), (Jobling, S.A., and Gehrke, L., (1987), Nature, 325:622-625;
Tobacco mosaic virus leader (TMV), (Gallie, D.R. et al., (1989), Molecular Biology of RNA, pages 237-256; and
Maize Chlorotic Mottle Virus leader (MCMV) (Lommel, S.A. et al., (1991), Virology, 81:382-385. See also, Della-Cioppa et al., (1987), Plant Physiology, 84:965-968.

A plant terminator may be utilized in the expression cassette. See, Rosenberg et al., (1987), Gene, 56:125; Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639.

For tissue specific expression, the nucleotide sequences of the invention can be operably linked to tissue specific promoters. See, for example, US Application Serial No. 07/951,715 herein incorporated by reference.

It is recognized that the genes encoding the pesticidal proteins can be used to transform insect pathogenic organisms. Such organisms include Baculoviruses, fungi, protozoa, bacteria and nematodes.

The Bacillus strains of the invention may be used for protecting agricultural crops and products from pests. Alternatively, a gene encoding the pesticide may be introduced via a suitable vector into a microbial host, and said host applied to the environment or plants or animals. Microorganism hosts may be selected which are known to occupy the "phytosphere" (phylloplane, phyllosphere, rhizosphere, and/or rhizoplana) of one or more crops of interest. These microorganisms are selected so as to be capable of successfully competing in the particular environment with the wild-type microorganisms, provide for stable maintenance and expression of the gene expressing the polypeptide pesticide, and, desirably, provide for improved protection of the pesticide from environmental degradation and inactivation.

Such microorganisms include bacteria, algae, and fungi. Of particular interest are microorganisms, such as bacteria, e.g.,Pseudomonas, Erwinia, Sematia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc, and Alcaligenes; fungi, particularly yeast, e.g.,Saccharomyces, Cryptococcus, Kluyveromyces, Sporobolomyces, Rhodotorula, and Aureobasidium. Of particular interest are such phytosphere bacterial species as Pseudomonas syringae, Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacter xyli and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces rosues, S. odorus, Kluyveromyces veronae, and Aureobasidium pollulans. Of particular interest are the pigmented microorganisms.

A number of ways are available for introducing a gene expressing the pesticidal protein into the microorganism host under conditions which allow for stable maintenance and expression of the gene. For example, expression cassettes can be constructed which include the DNA constructs of interest operably linked with the transcriptional and translational regulatory signals for expression of the DNA constructs, and a DNA sequence homologous with a sequence in the host organism, whereby integration will occur, and/or a replication system which is functional in the host, whereby integration or stable maintenance will occur.

Transcriptional and translational regulatory signals include but are not limited to promoter, transcriptional initiation start site, operators, activators, enhancers, other regulatory elements, ribosomal binding sites, an initiation codon, termination signals, and the like. See, for example, US Patent 5,039,523; US Patent No. 4,853,331; EPO 0480762A2; Sambrook et al. supra; Molecular Cloning, a Laboratory Manual, Maniatis et al. (eds) Cold Spring Harbor Laboratory, Cold Spring Harbor, NY (1982); Advanced Bacterial Genetics, Davis et al. (eds.) Cold Spring Harbor Laboratory, Cold Spring Harbor, NY (1980); and the references cited therein.

Suitable host cells, where the pesticide-containing cells will be treated to prolong the activity of the toxin in the cell when the then treated cell is applied to the environment of the target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae and Nitrobacteraceae. Among eukaryotes are fungi, such as Phycomycetes and Ascomycetes, which includes yeast, such a Saccharomyces and Schizosaccharromyces; and Basidiomycetes yeast, such as Rhodotorula, Aureobasidium, Sporobolomyces, and the like.

Characteristics of particular interest in selecting a host cell for purposes of production include ease of introducing the protein gene into the host, availability of expression systems, efficiency of expression, stability of the protein in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., and the like. Specific organisms include Pseudomonas aeurginosa, Pseudomonas fluorescens, Saccharomyces cerevisiae, Bacillus thuringiensis, Escherichia coli, Bacillus subtilis, and the like.

General methods for employing the strains of the invention in pesticide control or in engineering other organisms as pesticidal agents are known in the art. See, for example US Patent No. 5,039,523 and EP 0480762A2.

The Bacillus strains of the invention or the microorganisms which have been genetically altered to contain the pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticides are produced by introducing a heterologous gene into a cellular host. Expression of the heterologous gene results, directly or indirectly, in the intracellular production and maintenance of the pesticide. These cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein.

The active ingredients of the present invention are normally applied in the form of compositions and can be applied to the crop area or plant to be treated, simultaneously or in succession, with other compounds. These compounds can be both fertilizers or micronutrient donors or other preparations that influence plant growth. They can also be selective herbicides, insecticides, fungicides, bactericides, nematicides, mollusicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

Preferred methods of applying an active ingredient of the present invention or an agrochemical composition of the present invention which contains at least one of the pesticidal proteins produced by the bacterial strains of the present invention are leaf application, seed coating and soil application. The number of applications and the rate of application depend on the intensity of infestation by the corresponding pest.

In one embodiment of the invention a Bacillus cereus microorganism has been isolated which is capable of killing Diabrotica virgifera virgifera, and Diabrotica lonsticomis barberi. The novel B. cereus strain AB78 has been deposited in the Agricultural Research Service, Patent Culture Collection (NRRL), Northern Regional Research Center, 1815 North Univeristy Street, Peoria, IL 61604, USA and given Accession No. NRRL B-21058.

A protein has been substantially purified from the B. cereus strain. Purification of the protein has been verified by SDS-PAGE and biological activity. The protein has a molecular weight of about 60 to about 100 kDa, particularly about 70 to about 90 kDa, more particularly about 80 kDa.

Amino-terminal sequencing has revealed the N-terminal amino-acid sequence to be: NH₂-Lys-Arg-Glu-Ile-Asp-Glu-Asp-Thr-Asp-Thr-Asx-Gly-Asp-Ser-Ile-Pro- (SEQ ID NO:8) where Asx represents either Asp or Asn. The entire amino acid sequence is given in SEQ ID NO:7.

An oligonuleotide probe for the region of the gene encoding amino acids 3-9 of the NH₂-terminus has been generated. The probe was synthesized based on the codon usage of a Bacillus thuringensis (Bt) δ-endotoxin gene. The nucleotide sequence of the oligonucleotide probe used for Southern hybridizations was as follows: 5'- GAA ATT GAT CAA GAT ACN GAT -3'    (SEQ ID NO:9) where N represents any base.

In addition, the DNA probe for the Bc AB78 VIP-1 gene described herein, permits the screening of any Bacillus strain or other organisms to determine whether the VIP-1 gene (or related gene) is naturally present or whether a particular transformed organism includes the VIP-1 gene.

The invention now being generally described, the same will be better understood by reference to the following detailed examples that are provided for the purpose of illustration and are not to be considered limiting of the invention unless so specified.

### EXPERIMENTAL

### Example 1. AB78 Isolation and Characterization

Bacillus cereus strain AB78 was isolated as a plate contaminant in the laboratory on T3 media (per liter: 3 g tryptone, 2 g tryptose, 1.5 g yeast extract, 0.05 M sodium phosphate (pH 6.8), and 0.005 g MnCl₂; Travers, R.S. 1983). AB78 gave significant activity against western corn rootworm. Antibiotic activity against gram-positive Bacillus spp. was also dernonstrated (Table 12).

**Table 12**

| Antibiotic activity of AB78 culture supernatant | | |
|---|---|---|
| Bacteria tested | AB78 | Zone of inhibition(cm) Streptomycin |
| E. coli | 0.0 | 3.0 |
| B. megaterium | 1.1 | 2.2 |
| B. mycoides | 1.3 | 2.1 |
| B. cereus CB | 1.0 | 2.0 |
| B. cereus 11950 | 1.3 | 2.1 |
| B. cereus 14579 | 1.0 | 2.4 |
| B. cereus AB78 | 0.0 | 2.2 |
| Bt var. isrealensis | 1.1 | 2.2 |
| Bt var. tenebrionis | 0.9 | 2.3 |

Morpholgical characteristics of AB78 are as follows:
Vegetative rods straight, 3.1-5.0 mm long and 0.5-2.0 mm wide. Cells with rounded ends, single in short chains. Single subterminal, cylindical-oval, endospore formed per cell. No parasporal crystal formed. Colonies opaque, erose, lobate and flat. No pigments produced. Cells motile. Flagella present.

Growth characteristics of AB78 are as follows:
Facultative anaerobe with optimum growth temperature of 21-30°C. Will grow at 15, 20,25,30 and 37°C. Will not grow above 40°C. Grows in 5-7% NaCl.

Table 13 provides the biochemical profile of AB78.

**Table 13**

| Biochemical characteristics of B. cereus strain AB78. | | | |
|---|---|---|---|
| Acid from L-arabinose | - | Methylene blue reoxidized | + |
| Gas from L-arabinose | - | Nitrate reduced | + |
| Acid from D-xylose | - | NO₃ reduced to NO₂ | + |
| Gas from D-xylose | - | VP | + |
| Acid from D-glucose | + | H₂O₂ decomposed | + |
| Gas from D-glucose | - | Indole | - |
| Acid from lactose | - | Tyrosine decomposed | + |
| Gas from lactose | - | Dihydroxiacetone | - |
| Acid from sucrose | - | Litmus milk acid | - |
| Gas from sucrose | - | Litmus milk coagulated | - |
| Acid from D-mannitol | | - Litmus milk alkaline | |
| Gas from D-mannitol | - | Litmus milk peptonized | - |
| Proprionate utilization | | + Litmus milk reduced | |
| Citrate utilization | + | Casein hydrolyzed | + |
| Hippurate hydrolysis | w | Starch hydrolyzed | + |
| Methylene blue reduced | + | Gelatin liquidified | + |
| | | Lecithinase produced | w |
| w= weak reaction | | | |

### Example 2. Bacterial Culture

A subculture of Bc strain AB78 was used to inoculate the following medium, known as TB broth:

| | | |
|---|---|---|
| Tryptone | 12 | g/l |
| Yeast Extract | 24 | g/l |
| Glycerol | 4 | ml/l |
| KH₂PO₄ | 2.1 | g/l |
| K₂HPO₄ | 14.7 | g/l |
| pH 7.4 | | |

The potassium phosphate was added to the autoclaved broth after cooling. Flasks were incubated at 30°C on a rotary shaker at 250 rpm for 24 h.-36 h.

The above procedure can be readily scaled up to large fermentors by procedures well known in the art.

During vegetative growth, usually 24-36 h. after starting the culture, AB78 bacteria were centrifuged from the culture supernatant. The culture supernatant containing the active protein was used in bioassays.

### Example 3. Insect Bioassays

B. cereus strain AB78 was tested against various insects as described below.
**Western, Northern and Southern corn rootworm, Diabrotica virgifera virgifera, D. longcornis barberi and D. undecempunctata howardi, respectively:,** dilutions were made of AB78 culture supernatant grown 24-36 h., mixed with molten artificial diet (Marrone et al. (1985) J. of Economic Entomology 78:290-293) and allowed to solidify. Solidified diet was cut and placed in dishes. Neonate larvae were placed on the diet and held at 30°C. Mortality was recorded after 6 days.

**E. coli clone bioassay: E. coli** was grown overnight in L-Amp100 at 37°C. Ten ml culture was sonicated 3X for 20 sec each. 500 ml of sonicated culture was added to molten western corn rootworm diet.

**Colorado potato beetle Leptinotarsa decemlineata:**-dilutions in Triton X-100 (to give final concentration of 0.1% TX-100) were made of AB78 culture supernatant grown 24-36 h. Five cm² potato leaf pieces were dipped into these dilutions, air dried, and placed on moistened filter paper in plastic dishes. Neonate larvae were placed on the leaf pieces and held at 30°C. Mortality was recorded after 3-5 days.

**Yellow mealworm, Tenebrio molitor:**- dilutions were made of AB78 culture supernatant grown 24-36 h., mixed with molten artificial diet (Bioserv #F9240) and allowed to solidify. Solidified diet was cut and placed in plastic dishes. Neonate larvae were placed on the diet and held at 30°C. Mortality was recorded after 6-8 days.

**European corn borer, black cutworm, tobacco budworm, tobacco hornworm and beet armyworm; Ostrinia nubilalis, Agrotis ipsilon, Heliothis virescens, Manduca sexta and Spodoptera exigua,** respectively: -dilutions, in TX-100 (to give final concentration of 0.1% TX-100), were made of AB78 culture supernatant grown 24-36 hrs. 100 ml was pipetted onto the surface of 18 cm² of solidified artifical diet (Bioserv #F9240) and allowed to air dry. Neonate larvae were then placed onto the surface of the diet and held at 30°C. Mortality was recorded after 3-6 days.

**Northern house mosquito, Culex pipiens:**-dilutions were made of AB78 culture supernatant grown 24-36 h. 100 ml was pipetted into 10 ml water in a 30 ml plastic cup. Third instar larvae were added to the water and held at room temperature. Mortality was recorded after 24-48 hours. The spectrum of entomocidal activity of AB78 is given in Table 14.

**Table 14**

| Activity of AB78 culture supernatant against various insect species | | |
|---|---|---|
| Insect species tested to date | Order | Activity |
| Western corn rootworm | | |
| (Diabrotica virgifera virgifera) | Col | +++ |

| Northern corn rootworm | | |
|---|---|---|
| (Diabrotica longicomis barberi) | Col | +++ |

| Southern corn rootworm | | |
|---|---|---|
| (Diabrotica undecimpunctata howardi) | Col | - |

| Colorado potato beetle | | |
|---|---|---|
| (Leptinotarsa decemlineata) | Col | - |

| Yellow mealworm | | |
|---|---|---|
| (Tenebrio molitor) | Col | - |

| European corn borer | | |
|---|---|---|
| (Ostrinia nubilalis) | Lep | - |

| Tobacco budworm | | |
|---|---|---|
| (Heliothis virescens) | Lep | - |

| Tobacco hornworm | | |
|---|---|---|
| (Manduca sexta) | Lep | - |

| Beet armyworm | | |
|---|---|---|
| (Spodoptera exigua) | Lep | - |

| Black cutworm | | |
|---|---|---|
| (Agrotis ipsilon) | Lep | - |

| Northern house mosquito | | |
|---|---|---|
| (Culex pipiens) | Dip | - |

The newly discovered B. cereus strain AB78 showed a significantly different spectrum of insecticidal activity as compared to known coleopteran active δ-endotoxins from Bt. In particular, AB78 showed more selective activity against beetles than known coleopteran-active Bt strains in that it was specifically active to Diabrotica spp. More specifically, it was most active against D. virgifera virgifera and D. longicornis barberi but not D. undecimpunctata howardi.

A number of Bacillus strains were bioassayed for activity during vegetative growth (Table 15) against western corn rootworm. The results demonstrate that AB78 is unique in that activity against western corn rootworm is not a general phenomenon.

**Table 15**

| Activity of culture supernatants from various Bacillus spp. against western com rootworm | |
|---|---|
| Bacillus strain | Percent WCRW mortality |
| B. cereus AB78 (Bat.1) | 100 |
| B. cereus AB78 (Bat.2) | 100 |
| B. cereus (Carolina Bio.) | 12 |
| B. cereus ATCC 11950 | 12 |
| B. cereus ATCC 14579 | 8 |
| B. mycoides (Carolina Bio.) | 30 |
| B. popilliae | 28 |
| B. thuringiensis HD135 | 41 |
| B. thuringiensis HD191 | 9 |
| B. thuringiensis GC91 | 4 |
| B. thuringiensis isrealensis | 24 |
| Water Control | 4 |

Specific activity of AB78 against western corn rootworm is provided in Table 16.

**Table 16**

| Activity of AB78 culture supernatant against neonate western corn rootworm | |
|---|---|
| Culture supernatant concentration (µl/ml) | Percent WCRW mortality |
| 100 | 100 |
| 25 | 87 |
| 10 | 80 |
| 5 | 40 |
| 2.5 | 20 |
| 1 | 6 |
| 0 | 0 |

The LC₅₀ was calculated to be 6.2 µl of culture supernatant per ml of western corn rootworm diet.

### Example 4. Isolation and Purification of Corn Rootworm Active Protein from AB78.

Culture media free of cells and debris was made to 70% saturation by the addition of solid ammonium sulfate i.e. (472 g/L). Dissolution was at room temperature followed by cooling in an ice bath and centrifugation at 10,000 x g for thirty minutes to pellet out the precipitated proteins.

The supernatant was discarded and the pellet was dissoved in 1/10 the original volume with 20 mM TRIS-HCl at pH 7.5.

The dissolved pellet was desalted either by dialysis in 20 mM TRIS HCl pH 7.5, or passing through a desalting column.

The desalted material was titrated to pH 3.5 with 20 mM sodium citrate pH 2.5. Following a thirty minute room temperature incubation the solution was centrifuged at 3000 X g for ten minutes. The supernatant at this stage contained the greatest amount of active protein.

Following neutralization of the pH to 7.0 the supernatant was applied to a Mono-Q, anion exchange, column equilibrated with 20 mM TRIS pH 7.5 at a flow rate of 300 mL/min. The column was develped with a stepwise and linear gradient employing 400 mM NaCl in 20 mM TRIS pH 7.5.

Bioassay of the column fractions and SDS-PAGE analysis were used to confirm the active fractions. SDS-PAGE analysis identified the biologically active protein as having a molecular weight in the range of 80 kDa.

### Example 5. Sequence Analysis of the Corn Rootworm Active Protein

The 80 kDa protein isolated by SDS-PAGE was transferred to PVDF membrane and was subjected to amino-terminal sequencing as performed by repetitive Edman cycles on the ABI 470 pulsed-liquid sequencer. Transfer was carried out in 10 mM CAPS buffer with 10% methanol pH 11.0 as follows: Incubation of the gel following electrophoresis was done in transfer buffer for five minutes.

ProBlott PVDF membrane was wetted with 100% MeOH briefly then equilibrated in transfer buffer.

The sandwich was arranged between foam sponges and filter paper squares with the configuration of Cathode-Gel-Membrane-Anode.

Transfer was performed at 70 V constant voltage for 1 hour.

Following transfer the membrane was rinsed with water and stained for two minutes with 0.25% Coomassie Blue R-250 in 50% MeOH.

Destaining was done with several rinses with 50% MeOH 40% water 10% acetic acid.

Following destaining the membrane was air dried prior to excision of the bands for sequence analysis. A BlottCartridge and appropriate cycles were utilized to achieve maximum efficiency and yield. Data analysis was performed using the model 610 Sequence Analysis software for identifying and quantifying the PTH-amino acid derivatives for each sequential cycle.

The N-terminal sequence was determined to be: NH₂-Lys-Arg-Glu-Ile-Asp-Glu-Asp-Thr-Asp-Thr-Asx-Gly-Asp-Ser-Ile-Pro- (SEQ ID NO:8) where Asx represents Asp or Asn.

### Example 6. Construction of DNA Probe

An oligonucleotide probe for the region of the gene encoding amino acids 3-9 of the N-terminal sequence (Example 5) was generated. The probe was synthesized based on the codon usage of a Bacillus thuringensis (Bt) δ-endotoxin gene. The nucleotide sequence 5'- GAA ATT GAT CAA GAT  ACN GAT -3'      (SEQ ID NO:9) was used as a probe in Southern hybridizations. The oligonucleotide was synthesized using standard procedures and equipment.

### Example 7. Isoelectric Point Determination of the Corn Rootworm Active Protein

Purified protein from step 5 of the purification process was analyzed on a 3-9 pI isoelectric focusing gel using the Phastgel electrophoresis system (Pharmacia). Standard operating procedures for the unit were followed for both the separation and silver staining development procedures. The pI was approximated at about 4.9.

### Example 8. PCR Data On AB78

PCR analysis (See, for example US patent Application Serial No. 08/008,006; and, Carozzi et al. (1991) Appl. Environ. Microbiol. 57(11):3057-3061, herein incorporated by reference.) was used to verify that the B. cereus strain AB78 did not contain any insecticidal crystal protein genes of B. thuringensis or B. sphaericus (Table 17).

**Table 17**

| Bacillus insecticidal crystal protein gene primers tested by PCR against AB78 DNA. | |
|---|---|
| Primers Tested | Product Produced |
| 2 sets specific for CryIIIA | Negative |
| CryIIIB | Negative |
| 2 sets specific for CrylA | Negative |
| CryIA(a) | Negative |
| CryIA(b) specific | Negative |
| CryIB | Negative |
| CryIC specific | Negative |
| CryIE specific | Negative |
| 2 sets specific for B. sphaericus | Negative |
| 2 sets specific for CryIV | Negative |
| Bacillus control (PI-PLC) | Positive |

### Example 9. Cosmid Cloning of Total DNA from B. cereus Strain AB78

The VIP-1 gene was cloned from total DNA prepared from strain AB78 as follows:

Isolation of AB78 DNA was as follows:
1. Grow bacteria in 10 ml L-broth overnight. (Use 50 ml sterile centrifuge tube)
2. Add 25 ml of fresh L-broth and ampicillin (30 mg/ml).
3. Grow cells 2-6 h. at 30°C with shaking.
4. Spin cells in a 50 ml polypropylene orange cap tube in IEC benchtop clinical centrifuge at 3/4 speed.
5. Resuspend cell pellet in 10 ml TES.
6. Add 30 mg lyzozyme and incubate 2 hrs at 37°C.
7. Add 200 ml 20% SDS and 400 ml Proteinase K (20 mg/ml). Incubate at 37°C.
8. Add 200 ml fresh Proteinase K. Incubate 1 hr. at 55°C. Add 5 ml TES (TES = 50 mM tirs pH 8.0, 100mM EDTA, 15 mM NaCl) to make 15 ml final volume.
9. Phenol extract twice (10 ml phenol, spin at room temperature at 3/4 speed in an IEC benchtop clinical centrifuge). Transfer supernatant (top) to a clean tube with a wide bore pipet.
10. Extract once with 1:1 vol. phenol:chloroform/isoamyl alcohol (24:1 ratio).
11. Precipitate DNA with an equal volume of cold isopropanol; Centrifuge to pellet DNA.
12. Resuspend pellet in 5 ml TE.
13. Precipitate DNA with 0.5 ml 3M NaOAc pH 5.2 and 11 ml 95% ethanol. Place at -20°C for 2 h.
14. "Hook" DNA from tube with a plastic loop, transfer to a microfuge tube, spin, pipet off excess ethanol, dry in vacuo.
15. Resuspend in 0.5 ml TE. Incubate 90 min. at 65°C to help get DNA back into solution.
16. Determine concentration using standard procedures.

### Cosmid Cloning of AB78

All procedures, unless indicated otherwise, were performed according to Stratagene Protocol, Supercos 1 Instruction Manual, Cat. No. 251301.

Generally, the steps were as follows:
A. Sau 3A Partial Digestion of the AB78 DNA.
B. Preparation of Vector DNA
C. Ligation and packaging of DNA
D. Titering the cosmid library
   1. Start a culture of HB 101 cells by placing 50 ml of an overnight culture in 5 mls of TB with 0.2% maltose. Incubate 3.5 hrs. at 37°C.
   2. Spin out cells and resuspend in 0.5 mls 10 mM MgSO₄.
   3. Add together:
      100 ml cells
      100 ml diluted packaging mixture
      100 ml 10 mM MgSO₄
      30 ml TB
   4. Adsorb at room temperature for 30 minutes with no shaking.
   5. Add 1 ml TB and mix gently. Incubate 30 minutes at 37°C.
   6. Plate 200 ml onto L-amp plates. Incubate at 37°C overnight.

At least 400 cosmid clones were screened for activity against western corn rootworm as described in Example 3. DNA from 5 active clones and 5 non-active clones were used in Southern hybridizations. Results demonstrated that hybridization using the above described oligonucleotide probe correlated with western corn rootworm activity (Table 18).

Cosmid clones P3-12 and P5-4 have been deposited with the Agricultural Research Service Patent Culture Collecton (NRRL) and given accession nos. B-21061 and B-21059 respectively.

**Table 18**

| **Activity of AB78 cosmid clones against western corn rootworm.** | |
|---|---|
| Clone | Mean percent mortality (N=4) |
| Clones which hybridize with probe | |
| | |
| P1-73 | 47 |
| P1-83 | 64 |
| P2-2 | 69 |
| P3-12 | 85 |
| P5-4 | 97 |
| | |

| Clones which do not hybridize with probe | |
|---|---|
| | |
| P1-2 | 5 |
| P3-8 | 4 |
| P3-9 | 12 |
| P3-18 | 0 |
| P4-6 | 9 |

### Example 10. Identification of a 6 kb region active against western corn rootworm.

DNA from P3-12 was partially digested with restriction enzyme *Sau* 3A, and ligated into the E. coli vector pUC 19 and transformed into E. coli. A DNA probe specific for the 80 kDa protein was synthesized by PCR amplification of a portion of P3-12 DNA. The oligonucleotides MK113 and MK117, which hybridize to portions of VIP-1, were synthesized using the partial amino acid sequence of the 80 kDa protein. Plasmid subclones were identified by colony hybridization to the PCR probe, and tested for activity against western corn rootworm. One such clone, PL2, hybridizes to the PCR fragment, and is active against western corn rootworm by the assay previously described.

A 6 kb *Cla* I restriction fragment from PL2 was cloned into the *Sma* I site of the *E.coli*-Bacillus shuttle vector pHT 3101 (Lereclus, D. *et al*, 1989, FEMS Microbiology Letters **60**:211-218) to yield pCIB6201. This construct confers anti-western corn rootworm activity upon both Bacillus and *E.coli* strains, in either orientation. pCIB6022 contains this same 6 kb *Cla* I fragment in pBluescript SK(+) (Stratagene), produces equivalent VIP-1 protein (by western blot), and is also active against western corn rootworm.

The nucleotide sequence of pCIP6022 was determined by the dideoxy termination method of Sanger et al., Proc. Natl. Acad. Sci. USA, 74:5463-5467 (1977), using PRISM Ready Reaction Dye Deoxy Terminator Cycle Sequencing Kits and PRISM Sequenase® Terminator Double-Stranded DNA Sequencing Kit and analyzed on AB1 373 automatic sequencer. The sequence is given in SEQ ID NO:1. pCIB6022 was deposited with the Agricultural Research Service, Patent Culture Collection, (NRRL), Northern Regional Research Center, 1815 North University Street, Peoria, Illinois 61604, USA, and given the Accession No. NRRL B-21222.

### Example 11. Functional dissection of the VIP-1 DNA region.

To confirm that the VIP-1 open reading frame (ORF) is necessary for insecticidal activity a translational frameshift mutation was created in the gene. The restriction enzyme Bgl II recognizes a unique site located 1758 bp into the coding region of VIP-1. pCIB6201 was digested with Bgl II, and the single-stranded ends filled-in with DNA polymerase (Klenow fragmnent) and dNTPS. The plasmid was re-ligated and transformed into E. coli. The resulting plasmid, pCIB6203, contains a four nucleotide insertion in the coding region of VIP-1. pCIB6203 does not confer insecticidal activity, confirming that VIP-1 is an essential component of western corn rootworm activity.

To further define the region necessary to encode VIP-1, subclones of the VIP-1 and VIP-2 (auxiliary protein) region were constructed and tested for their ability to complement the mutation in pCIB6203. pCIB6023 contains the 3.7kb Xba I-EcoRV fragment in pBluescript SK(+) (Stratagene). Western blot analysis indicates that pCIB6023 produces VIP-1 protein of equal size and quantity as clones PL2 and pCIB 6022. pCIB6023 contains the entire gene for the 80kd protein. pCIB6023 was deposited with the Agricultural Research Service, Patent Culture Collection, (NRRL), Northern Regional Research Center, 1815 North University Street, Peoria, Illinois 61604, USA, and given the Accession No. NRRL B-21223.

pCIB6023 shows some western corn rootworm activity. However, the level of activity is less than the activity of pCIB6022. A mixture of cells containing pCIB6203 (VEP-1-mutated, and VIP-2) and cells containing pCIB6023(only VIP-1) shows high activity against western corn rootworm. Thus, pCIB6023 must produce functional VIP-1 gene product, and pCIB6203 must produce a functional VIP-2 gene product. These results suggest a requirement for additional gene product(s) from the VIP-2 region, in combination with VIP-1, to confer maximal western corn rootworm activity. See Table 19.

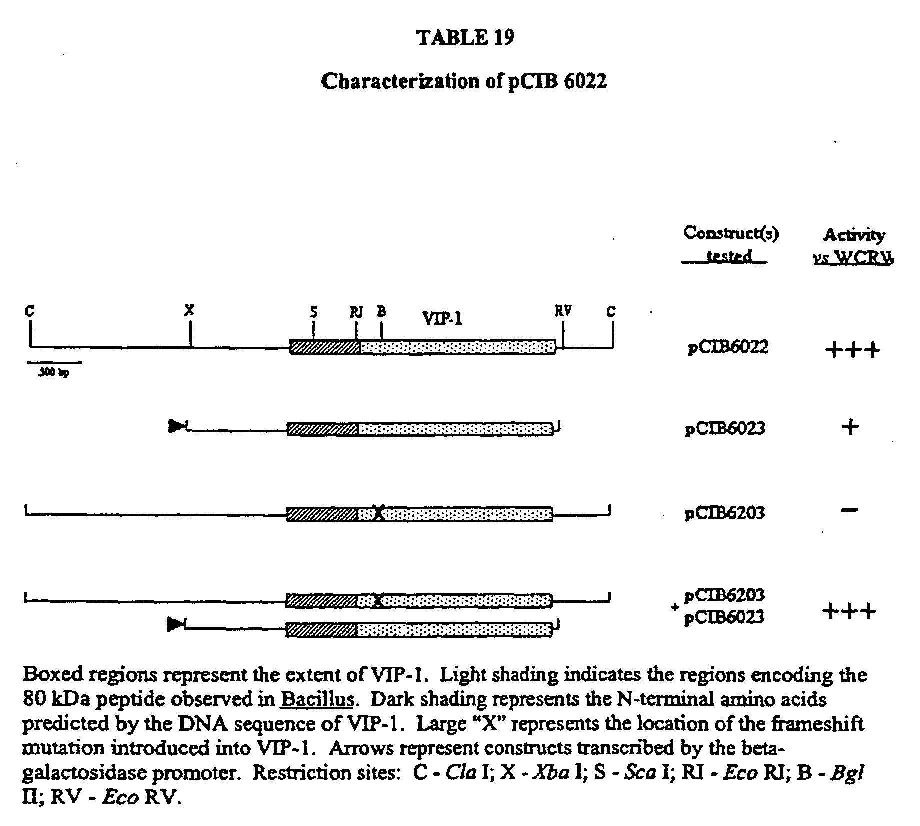

### Example 12. AB78 Antibody Production

Antibody production was initiated in 2 Lewis rats to allow for both the possibility of moving to production of hybridoma cell lines and also to produce enough serum for limited screening of cDNA library. Another factor was the very limited amount of antigen available and the fact that it could only be produced to purity by PAGE and subsequent electrotransfer to nitrocellulose.

Due to the limited availability of antigen on nitrocellulose, the nitrocellulose was emulsified in DMSO and injected into the hind footpads of the animals to elicit B-cell production in the popliteal lymph nodes just upstream. A strong reacting serum was produced by western analysis within the first production bleed. Several subsequent injections and bleeds produced enough serum to accomplish all of the screening required.

Hybridoma production with one of the rats was then initiated. The popliteal lymph node was excised, macerated, and the resulting cells fused with mouise myeloma P3x63Ag8.653. Subsequent cell screening was accomplished as described below. Four initial wells were selected which gave the highest emulsified antigen reaction to be moved to limited dilution cloning. An additional 10 wells were chosen for expansion and cryoperservation.

### Procedure to Emulsify AB78 on nitrocellulose in DMSO for ELISA screening:

After electrotransfer of AB78 samples run on PAGE to nitrocellulose, the reversible strain Ponceaus is used to visualize all protein transferred. The band corresponding to AB78 toxin, previously identified and N-terminal sequenced, is identified and excised from nitrocellulose. Each band is approximately 1mmx5mm in size to minimize the amount of nitrocellulose emulsified. A single band is placed in a microfuge tube with 250ul of DMSO and macerated using a plastic pestle (Kontes, Vineland, NJ). To aid in emulsification, the DMSO mixture is heated for 2-3 minutes at 37°C-45°C. Some further maceration might be necessary following heating; however, all of the nitrocellulose should be emulsified. Once the AB78 is emulsified, the sample is placed on ice. In preparation for microtiter plate coating with the emulsified antigen, the sample must be diluted in borate buffered saline as follows: 1:5, 1:10, 1:15, 1:20, 1:30, 1:50, 1:100, and 0. The coating antigen must be prepared fresh immediately prior to use.

ELISA protocol:
1. Coat with AB78/DMSO in BBS. Incubate overnight at 4°C.
2. Wash plate 3X with 1X ELISA wash buffer.
3. Block (1% BSA & 0.05% Tween 20 in PBS) for 30 minutes at Room Temperature.
4. Wash plate 3X with 1X ELISA wash buffer.
5. Add Rat Serum. Incubate 1.5 hours at 37°C.
6. Wash plate 3X with 1X ELISA wash buffer.
7. Add Goat anti-Rat at a conc. of 2ug/ml in ELISA diluent. Incubate 1 hr. at 37°C.
8. Wash plate 3X with 1X ELISA wash buffer.
9. Add Rabbit anti-Goat Alkaline Phosphatase at 2ug/ml in ELISA diluent. Incubate 1Hr. at 37°C.
10. Wash 3X with 1X ELISA wash buffer.
11. Add Substrate. Incubate 30 minutes at Room Temperature.
12. Stop with 3N NaOH after 30 minutes.

### Example 13. Activation of insecticidal activity of non-active Bt strains with AB78 VIP clones.

Adding pCIB6203 together with culture supernatant from a Bt strain GC91 produces 100% mortality in Diabrotica virgifera virgifera. Neither pCIB6203 nor GC91 is active on Diabrotica virgifera virgifera by itself. Data are shown below:

| Test material | Percent Diabrotica mortality |
|---|---|
| pCD36203 | 0 |
| GC91 | 16 |
| pCIB6203 + GC91 | 100 |
| Control | 0 |

### Example 14. Isolation and Biological Activity of B.cereus AB81.

A second B. cereus strain, designated AB81, was isolated from grain bin dust samples by standard methodologies. A subculture of AB81 was grown and prepared for bioassay as described in Example 2. Biological activity was evaluated as described in Example 3. The results are as follows:

| Insect species tested | Percent Mortality |
|---|---|
| Ostrinia nubilalis | 0 |
| Agrotis ipsilon | 0 |
| Diabrotica virgifera virgifera | 55 |

### Example 15. Isolation and Biological Activity of B. thuringiensis AB6.

A B. thuringiensis strain, designated AB6, was isolated from grain bin dust samples by standard methods known in the art. A subculture of AB6 was grown and prepared for bioassay as described in Example 2. Half of the sample was autoclaved 15 minutes to test for the presence of β-exotoxin.

Biological activity was evaluated as described in Example 3. The results are as follows:

| Insect species tested | Percent Mortality |
|---|---|
| Qstrinia nubilalis | 0 |
| Agrotis ipsilon | 100 |
| Agrotis ipsilon (autoclaved sample) | 0 |
| Diabrotica virgifera virgifera | 0 |

Strain AB6 has been deposited in the Agricultural Research Service, Patent Culture Collection (NRRL), Northern Regional Research Center, 1815 North University Street, Peoria, Illinois 61604, USA, and given Accession No. NRRL B-21060.

### Example 16. Isolation and Biological characterization of B. thuringiensis AB88.

A Bt strain, designated AB88, was isolated from grain bin dust samples by standard methodologies. A subculture of AB88 was grown and prepared for bioassay as described in Example 2. Half of the sample was autoclaved 15 minutes to test for the presence of β-exotoxin. Biological activity was evaluated against a number of insect species as described in Example 3. The results are as follows:

| | | Percent mortality of culture supernatant | |
|---|---|---|---|
| Insect species tested | Order | Non-autoclaved | Autoclaved |
| Agrotis ipsilon | Lepidoptera | 100 | 5 |
| Ostrinia nubilalis | Lepidoptera | 100 | 0 |
| Spodoptera frugiperda | Lepidoptera | 100 | 4 |
| Helicoverpa zea | Lepidoptera | 100 | 12 |
| Heliothis virescens | Lepidoptera | 100 | 12 |
| Leptinotarsa | | | |
| decemlineata | Coleoptera | 0 | 0 |
| Diabrotica virgifera virgifera | Coleoptera | 0 | 5 |

Delta-endotoxin crystals were purified from strain AB88 by standard methodologies. No activity from pure crystals was observed when bioassayed against Agrotis ipsilon.

### Example 17. Purification of VIPs from Strain AB88:

Bacterial liquid culture was grown overnight at 30°C in TB media. Cells were spun out and the supernatant kept. Proteins were precipitated with ammonium sulfate (70% saturation), centrifuged and the pellet kept. The pellet was resuspended in the original volume of 20 mM Tris pH 7.5 and dialyzed against the same buffer. AB88 dialysate was more turbid than comparable material from AB78. AB88 proteins have been separated by several different methods following clarification including isoelectric focusing (Rotofor, BioRad, Hercules, CA), precipitation at pH 4.5, ion-exchange chromotography, size exclusion chromatography and ultrafiltration.

European Corn Borer-active protein remained in the pellet obtained by pH 4.5 precipitation of dialysate. When preparative IEF was done on the dialysate using pH 3-10 ampholytes, ECB insecticidal activity was found in all fractions with pH of 7 or greater. SDS-PAGE of these fractions showed protein bands of MW ∼60 kDa and ∼80 kDa. The 60 kDa and 80 kDa bands were separated by anion exchange HPLC on a Poros-Q column (PerSeptive Biosystems, Cambridge, MA). N-terminal sequence was obtained from two fractions containing proteins of slightly differing MW, but both of approximately 60 kDa in size. The sequences obtained were similar to each other and to some δ-endotoxins.
- anion exchange fraction 23 (smaller):: xEPFVSAxxxQxxx (SEQ ID NO:10)
- anion exchange fraction 28 (larger):: xEYENVEPFVSAx (SEQ ID NO:11)

When the (active) pH 4.5 pellet was further separated by anion exchange on a Poros-Q column, activity was found only in fractions with a major band of ∼60 kDa.

Black Cutworm-active protein also remained in the pellet when AB88 dialysate was brought down to pH 4.5. In preparative IEF using pH 3-10 ampholytes, actyivity was not found in the ECB-active IEF fractions; instead, it was highest in a fraction of pH 4.5-5.0. Its major components have molecular weights of ∼35 and ∼80 kDa.

The pH 4.5 pellet was separated by anion exchange HPLC to yield fractions containing only the 35 kDa material and fractions containing both 35 kDa and 80 kDa bands.

### Example 18. Characterization of AB88 VIP.

Fractions containing the various lepidopteran active vegetative proteins were generated as described in Example 17. Analysis of active fractions demonstrates that different VIP's are responsible for the different lepidopteran species activity.

The Agrotis ipsilon activity is due to an 80 kDa and or a 35 kDa protein either delivered singly or in combination. These proteins are not related to any δ-endotoxins from Bt as evidenced by the lack of sequence homology of known Bt δ-endotoxin sequences. Also, these proteins are not found in the AB88 δ-endotoxin crystal. N-terminal sequences of the major δ-endotoxin proteins were compared with the N-terminal sequences of the 80 kDa and 35 kDa VIP and reveal no sequence homology. A summary of the results follows:

| Agrotis VIP N-terminal sequences | N-terminal sequence of major δ-endotoxin proteins |
|---|---|
| | 130 kDa MDNNPNINE (SEQ ID NO:14) |
| | |
| 80 kDa MNKNNTKLPTRALP (SEQ ID NO:12) | 80 kDa MDNNPNINE (SEQ ID NO:15) |
| | |
| | 60 kDa MNVLNSGRTTI (SEQ ID NO:16) |
| 35 kDa ALSENTGKDGGYIVP (SEQ ID NO:13) | |

The Ostrinia nubilalis activity is due to a 60 kDa VIP and the Spodoptera frugiperda activity is due to a VIP of unknown size.
Bacillus thuringiensis strain AB88 has been deposited in the Agricultural Research Service, Patent Culture Collection (NRRL), Northern Regional Research Center, 1815 North University Street, Peoria, Ilinois 61604, USA and given the Accession No. NRRL B-21225.

### Example 19. Isolation and Biological Activity of Other Bacillus sp.

Other Bacillus species have been isolated which produce proteins with insecticidal activity during vegetative growth. These strains were isolated from environmental samples by standard methodologies. Isolates were prepared for bioassay and assayed as described in Examples 2 and 3 respectively. Isolates which produced insecticidal proteins during vegetative growth with activity against Agrotis ipsilon in the bioassay are tabulated below.

| Presence of δ-endotoxin | | |
|---|---|---|
| Bacillus isolate | crystal | Percent mortality |
| AB6 | + | 100 |
| AB53 | - | 80 |
| AB88 | + | 100 |
| AB195 | - | 60 |
| AB211 | - | 70 |
| AB217 | - | 83 |
| AB272 | - | 80 |
| AB279 | - | 70 |
| AB289 | + | 100 |
| AB292 | + | 80 |
| AB294 | - | 100 |
| AB300 | - | 80 |
| AB359 | - | 100 |

Isolates AB289, AB294 and AB359 have been deposited in the Agricultural Research Service, Patent Culture Collection (NRRL), Northern Regional Research Center, 1815 North University Street, Peoria Il 61604, USA and given the Accession Numbers NRRL B-21227, NRRL B-21229, and NRRL B-21226 respectively.

Bacillus isolates which produce insecticidal proteins during vegetative growth with activity against Diabrotica virgifera virgifera are tabulated below.

| Presence of δ-endotoxin | | |
|---|---|---|
| Bacillus isolate | crystal | Percent mortality |
| AB52 | - | 50 |
| AB59 | - | 71 |
| AB68 | + | 60 |
| AB78 | - | 100 |
| AB122 | - | 57 |
| AB218 | - | 64 |
| AB256 | - | 64 |

Isolates AB59 and AB256 have been deposited in the Agricultural Research Service, Patent Culture Collection (NRRL), Northern Regional Research Center, 1815 North University Street, Peoria Illinois 61604, USA, and given the Accession Numbers NRRL B-21228 and B-21230, respectively.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The following deposits have been made at Agricultural Research Service, Patent Culture Collection (NRRL), Northern Regional Research Center, 1815 North University Street, Peoria, Illinois 61604, U.S.A.:

| | | |
|---|---|---|
| 1. | E. coli PL2 | Accession No. NRRL B-21221 |
| 2. | E. coli pCIB 6022 | Accession No. NRRL B-21222 |
| 3. | E. coli pCIB 6023 | Accession No. NRRL B-21223 |
| 4. | Bacillus thuringiensis HD73-78VIP | Accession No. NRRL B-21224 |
| 5. | Bacillus thuringiensis AB88 | Accession No. NRRL B-21225 |
| 6. | Bacillus thuringiensis AB359 | Accession No. NRRL B-21226 |
| 7. | Bacillus thuringiensis AB289 | Accession No. NRRL B-21227 |
| 8. | Bacillus sp. AB59 | Accession No. NRRL 13-21228 |
| 9. | Bacillus sp. AB294 | Accession No. NRRL B-21229 |
| 10. | Bacillus sp. AB256 | Accession No. NRRL B-21230 |
| 11. | E. coli P5-4 | Accession No. NRRL B-21059 |
| 12. | E. coli P3-12 | Accession No. NRRL B-21061 |
| 13. | Bacillus cereus AB78 | Accession No. NRRL B-21058 |
| 14. | Bacillus thuringiensis AB6 | Accession No. NRRL B-21060 |

Although the foregoing invention has been described in some detail by way of illustration and example for purposes of clarity of understanding, it will be obvious that certain changes and modifications may be practiced within the scope of the appended claims.

## Claims

1. A substantially purified Bacillus strain which produces a pesticidal protein during vegetative growth.

2. The Bacillus strain of claim 1 wherein said Bacillus is selected from a Bacillus species listed in Table 11.

3. The Bacillus strain of claim 1 wherein said protein is capable of killing pests selected from insects, fungi, bacteria, nematodes, mites, ticks, protozoan pathogens, animal parasites, and the like.

4. The Bacillus strain of claim 3, wherein said protein is capable of killing insects selected from orders Coleoptera, Diptera, Hymenoptera, Lepidoptera, Homoptera, Hemiptera, Orthroptera, Thysanoptera, Dennaptera, Isoptera, Mallophaga, Anoplura, Siphonaptera, or Trichoptera.

5. The Bacillus strain of claim 4, wherein said coleopteran species is a Diabrotica.

6. The Bacillus strain of claim 5, wherein said Diabrotica is Diabrotica virgifera virgifera or Diabrotica longicornis barberi.

7. The Bacillus strain of claim 4, wherein said lepidopteran species is an Agrotis.

8. The Bacillus strain of claim 7, wherein said Agrotis is Agrotis ipsilon.

9. The Bacillus strain of claim 2, wherein said Bacillus is Bacillus cereus.

10. The Bacillus strain of claim 9, wherein said Bacillus cereus is Bacillus cereus having Accession No. NRRL B-21058.

11. The Bacillus strain of claim 2, wherein said Bacillus is Bacillus thuringensis.

12. The Bacillus strain of claim 11, wherein said Bacillus thuringensis is Bacillus thuringensis having Accession No. NRRL B-21060.

13. The Bacillus strain of claim 2, wherein said protein has a molecular weight of 30 kDa or greater.

14. The Bacillus strain of claim 13, wherein said protein has a molecular weight of about 60 to about 100 kDa.

15. The Bacillus strain of claim 14, wherein said protein has a molecular weight of about 80 kDa.

16. The Bacillus strain of claim 15, wherein said protein has the sequence given in SEQ. ID. NO:7.

17. The Bacillus strain of claim 14, wherein said protein has a molecular weight of about 100 kDa.

18. The Bacillus strain of claim 17, wherein said protein has the sequence given in SEQ. ID. NO:5.

19. A substantially pure pesticidal protein isolatable during the vegetative growth phase of Bacillus spp. or analogs and active fragments thereof.

20. The pesticidal protein of claim 19 wherein said Bacillus is selected from a Bacillus species listed in Table 11.

21. The pesticidal protein of claim 20, wherein said insects are selected from orders Coleoptera, Diptera, Hymenoptera, Lepidoptera, Homoptera, Hemiptera, Orthroptera, Thysanoptera, Dermaptera, Isoptera, Mallophaga, Anoplura, Siphonaptera, or Trichoptera.

22. The pesticidal protein of claim 21, wherein said coleopteran species is a Diabrotica.

23. The pesticidal protein of claim 22, wherein said Diabrotica is Diabrotica virgifera virgifera or Diabrotica longicornis barberi.

24. The pesticidal protein of claim 21, wherein said lepidopteran species is an Agrotis.

25. The pesticidal protein of claim 24, wherein said Agrotis is Agrotis ipsilon.

26. The pesticidal protein of claim 19, wherein said Bacillus is Bacillus cereus.

27. The pesticidal protein of claim 26, wherein said Bacillus cereus is Bacillus cereus having Accession No. B-21058.

28. The pesticidal protein of claim 19, wherein said Bacillus is Bacillus thuringensis.

29. The pesticidal protein of claim 28, wherein said Bacillus thuringensis is Bacillus thuringensis selected from Accession Numbers NRRL B-21060, NRRL B-21224, NRRL B-21225, NRRL B-21226 and NRRL B-21227.

30. The pesticidal protein of claim 19, wherein said protein has a molecular weight of 30 kDa or greater.

31. The pesticidal protein of claim 30, wherein said protein has a molecular weight of about 60 to about 100 kDa.

32. The pesticidal protein of claim 31, wherein said protein has a molecular weight of about 80 kDa.

33. The pesticidal protein of claim 32, wherein said protein has the sequence given in SEQ ID NO:7.

34. The pesticidal protein of claim 31, wherein said protein has the sequence given in SEQ ID NO:5.

35. The pesticidal protein of claim 19, wherein said protein comprises an N-terminal sequence as set forth in SEQ ID NOS:10 or 11.

36. A substantially pure nucleotide sequence which encodes the protein of claim 19.

37. A substantially pure nucleotide sequence which encodes the protein of claim 33.

38. A substantially pure nucleotide sequence which encodes the protein of claim 34.

39. A substantially pure nucleotide sequence which encodes the protein of claim 35.

40. The nucleotide sequence of claim 36, wherein said sequence has been optimized for expression in a plant.

41. The nucleotide sequence of claim 40, wherein said plant is selected from maize, soybean, cotton, wheat, sunflower, tomato, potato, and oilseed rape.

42. The nucleotide sequence of claim 37, wherein said sequence has been optimized for expression in a plant.

43. The nucleotide sequence of claim 42, wherein said plant is selected from maize, soybean, cotton, wheat, sunflower, tomato, potato, and oilseed rape.

44. The nucleotide sequence of claim 38, wherein said sequence has been optimized for expression in a plant.

45. The nucleotide sequence of claim 44, wherein said sequence is set forth in SEQ ID NO: 18.

46. The nucleotide sequence of claim 39, wherein said sequence has been optimized for expression in a plant.

47. The nucleotide sequence of claim 46, wherein said sequence is set forth in SEQ ID NO: 17.

48. The nucleotide sequence of claim 36, wherein said sequence has been optimized for expression in a microorganism.

49. The nucleotide sequence of claim 48, wherein said microorganism is selected from Bacillus, Pseudomonas, Saccharomyces, Clavibacter, Erwinia, Serratia, Klebsiella, Xanthomonas, Streptomyces. Agrobacterium, insect pathogenic viruses, fungi, protozoans and nematodes.

50. A plant which has been stably transformed with the nucleotide sequence of any one of claims 36-47

51. The plant of claim 48, wherein said plant is a maize plant.

52. The nucleotide sequence of claim 36, wherein said sequence is essentially the sequence of E. coli clone P5-4 having Accession No. NRRL B-21059.

53. The nucleotide sequence of claim 36, wherein said sequence is essentially the sequence of E. coli clone P3-12 having Accession No. NRRL B-21061.

54. The nucleotide sequence of claim 36, wherein said sequence is contained in E. coli clone pCIB 6022 having Accession No. NRRL B-21222.

55. The nucleotide sequence of claim 54 wherein said sequence is given as VIP-1 in SEQ ID NO:1.

56. An auxiliary protein which enhances the pesticidal activity of a pesticidal protein.

57. The auxiliary protein of claim 56 wherein said pesticidal protein is from Bacillus.

58. The auxiliary protein of claim 57 wherein said pesticidal protein is from B. cereus.

59. The auxiliary protein of claim 58 wherein said pesticidal protein is from strain AB78.

60. The auxiliary protein of claim 56 wherein said auxiliary protein is from Bacillus.

61. The auxiliary protein of claim 60 wherein said auxiliary protein is from B. cereus.

62. The auxiliary protein of claim 61 wherein said auxiliary protein is from strain AB78.

63. A substantially pure nucleotide sequence which encodes the auxiliary protein of any one of claims 56, 60, 61, and 62.

64. The nucleotide sequence of claim 63 wherein said sequence is contained in E. coli clone pCIB6022 having Accession No. NRRL B-21222.

65. The Bacillus strain of claim 1 wherein said strain is AB88 having Accession No. NRRL B-21225.

66. The Bacillus strain of claim 1 wherein said strain is AB289 having Accession No. NRRL B-21227.

67. The Bacillus strain of claim 1 wherein said strain is AB294 having Accession No. NRRL B-21229.

68. The Bacillus strain of claim 1 wherein said strain is AB359 having Accession No. NRRL B-21226.

69. The Bacillus strain of claim 1 wherein said strain is AB59 having Accession No. NRRL B-21228.

70. The Bacillus strain of claim 1 wherein said strain is AB256 having Accession No. NRRL B-21230.
